(19) **Europäisches Patentamt**
**European Patent Office**
**Office européen des brevets**

(11) **EP 3 078 970 A1**

(12) **EUROPEAN PATENT APPLICATION**

(43) Date of publication:
**12.10.2016 Bulletin 2016/41**

(51) Int Cl.:
**G01N 33/68** (2006.01)     **G06F 19/24** (2011.01)

(21) Application number: **15162685.0**

(22) Date of filing: **07.04.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(71) Applicants:
• **Université d'Angers**
  **49000 Angers (FR)**
• **Centre Hospitalier Universitaire
  d'Angers
  49100 Angers (FR)**
• **Centre Hospitalier Universitaire De Nantes
  44093 Nantes Cedex 1 (FR)**

• **Assistance Publique-Hôpitaux de Paris (APHP)
  75001 Paris (FR)**
• **Université Paris Diderot - Paris 7
  75205 Paris Cedex 13 (FR)**

(72) Inventors:
• **Calès, Paul**
  **49240 Avrillé (FR)**
• **Sacher-Huvelin, Sylvie**
  **44700 Orvault (FR)**
• **Galmiche, Jean-Paul**
  **76000 Rouen (FR)**
• **Valla, Dominique**
  **92270 Bois Colombes (FR)**

(74) Representative: **Icosa**
  **83 avenue Denfert-Rochereau
  75014 Paris (FR)**

(54) **NON-INVASIVE METHOD FOR ASSESSING THE PRESENCE AND SEVERITY OF ESOPHAGEAL VARICES**

(57)     The present invention relates to a non-invasive method for assessing the presence and/or severity of varices selected from gastric and esophageal varices in a liver disease patient, wherein said method comprises:
(a) carrying out a non-invasive test for assessing the severity of a hepatic lesion or disorder, wherein said non-invasive test results in a value, and
(b) comparing the value obtained at step (a) with cut-offs of said non-invasive test for assessing the presence and/or severity of varices selected from gastric and esophageal varices.

EP 3 078 970 A1

**Description**

**FIELD OF INVENTION**

The present invention relates to the assessment of the presence and/or severity of varices, including esophageal varices and gastric varices, in particular to the detection of large esophageal varices. More specifically, the present invention relates to a non-invasive method comprising measuring blood markers and optionally recovering data from an endoscopic capsule for assessing the presence and/or severity of esophageal or gastric varices.

**BACKGROUND OF INVENTION**

The majority of patients who succumb to fibrosis or cirrhosis dies due to complications of increased portal venous pressure, including variceal hemorrhage, ascites, hepatic encephalopathy, and the like. Indeed, severe fibrosis, especially cirrhosis, induces portal hypertension which, above a portal pressure level of 10 mmHg, provokes esophageal varices. Bleeding from ruptured esophageal varices is a major cause of mortality and economic burden in cirrhosis.

Primary prevention of first bleeding in large esophageal varices significantly reduces mortality. Therefore, the recommended work-up of cirrhotic patients includes systematic screening of large esophageal varices.

The gold standard method to diagnose large esophageal varices is upper gastro-intestinal endoscopy (UGIE). However, UGIE is somewhat limited by some constraints and notably the poor acceptance by the patients, due to the invasiveness of this method.

There is thus a need for non-invasive methods for diagnosing large esophageal varices.

Non-invasive diagnosis of large esophageal varices is currently not accurate enough to be adopted in practice. In particular, esophageal capsule endoscopy (ECE) presents a clinically significant probability of missed esophageal varices (i.e. false negative results) or of false positive results.

There is thus a need for a non-invasive method for diagnosing esophageal varices, which is more accurate than esophageal capsule endoscopy, and in particular which allows reducing missed esophageal varices.

Non-invasive diagnosis of liver fibrosis has gained considerable attention over the last 10 years as an alternative to liver biopsy. The first generation of simple blood fibrosis tests combined common indirect blood markers into a simple ratio, like APRI (Wai et al., Hepatology 2003) or FIB-4 (Sterling et al., Hepatology 2006). The second generation of calculated tests combine indirect and/or direct fibrosis markers by logistic regression, leading to a score, like Fibrotest (Imbert-Bismut et al., Lancet 2001), ELF score (Rosenberg et al., Gastroenterology 2004), FibroMeter™ (Cales et al., Hepatology 2005), Fibrospect™ (Patel et al., J Hepatology 2004), and Hepascore (Adams et al., Clin Chem 2005). For example, WO2005/116901 describes a non-invasive method for assessing the presence of a liver disease and its severity, by measuring levels of specific variables, including biological variables and clinical variables, and combining said variables into mathematical functions, generally binary mathematical function to provide a score result, often called "score of fibrosis".

There is currently a need for a non-invasive diagnostic test for directly assessing the presence and/or severity of varices.

WO2014/190170 describes a non-invasive test for assessing hepatic vein pressure gradient (HVPG) in cirrhotic patients, and suggests that this test may be used for assessing the absence of varices. However, the non-invasive test of WO2014/190170 presents the drawback of using blood markers without clinical potential, because these markers, while commonly used for research purpose, may not easily be used for clinical diagnosis, due either to a difficult implementation or to the cost of the measurement. Moreover, there is no experimental demonstration in WO2014/190170 that the described non-invasive test may efficiently be used for assessing the presence of esophageal varices. Furthermore, the non-invasive test of WO2014/190170 only results in two situations: either the patient shows a HVPG lower than 12 mmHg, and is diagnosed as not presenting esophageal varices, either the patient shows a HVPG of at least 12 mmHg, and an additional test is required for assessing the presence of esophageal varices (usually endoscopy).

The non-invasive test of WO2014/190170 was developed on cirrhotic patients, i.e. in patients already diagnosed with cirrhosis. However, the construction and performance evaluation of non-invasive tests of cirrhosis are limited by the characteristics of liver biopsy which is an imperfect gold standard. Therefore, a non-invasive test for assessing the presence of esophageal varices should ideally circumvent the intermediate step of cirrhosis diagnosis.

There is thus a need for a non-invasive method for diagnosing esophageal varices without the drawbacks of the non-invasive tests of the prior art.

In the present invention, the Applicants develop a non-invasive method for diagnosing esophageal varices in a patient with a liver disease (whether or not this patient was previously diagnosed as cirrhotic), wherein said method comprises performing a non-invasive diagnostic test for assessing the severity of a hepatic condition, using cut-offs for assessing the presence of varices instead of cut-offs for assessing the severity of a hepatic condition. In one embodiment, the method of the invention further comprises combining in a score blood markers, clinical markers, and data obtained by esophageal capsule endoscopy.

Experimental data obtained by the Applicant demonstrate that the non-invasive method of the invention may be used in any liver disease patient, and strongly reduces the number of missed esophageal varices as compared to ECE for example.

## SUMMARY

The present invention thus relates to a non-invasive method for assessing the presence and/or severity of varices, selected from gastric and esophageal varices in a liver disease patient, wherein said method comprises:

(a) carrying out a non-invasive test for assessing the severity of a hepatic lesion or disorder, wherein said non-invasive test results in a value, and
(b) comparing the value obtained at step (a) with cut-offs of said non-invasive test for assessing the presence and/or severity of varices, selected from gastric and esophageal varices.

In one embodiment, the method is for assessing the presence of large esophageal varices.
In one embodiment, said cut-offs are a NPV cut-off and a PPV cut-off, or a sensitivity cut-off and a specificity cut-off.
In one embodiment,

- a value obtained in step (a) below the NPV cut-off or below the sensitivity cut-off is indicative of the absence of varices, selected from gastric and esophageal varices, preferably of large esophageal varices, in the patient, and

- a value obtained in step (a) above the PPV cut-off or above the PPV cut-off is indicative of the presence of varices, selected from gastric and esophageal varices, preferably of large esophageal varices, in the patient.

In one embodiment, if the value obtained in step (a) is between the NPV cut-off and the PPV cut-off or between the sensitivity cut-off and the specificity cut-off, then the method further comprises the steps of:

(c) measuring at least one of the following variables from the subject:

- biomarkers,
- clinical data,
- binary markers,
- physical data from medical imaging or clinical measurement

(d) obtaining imaging data on varices status, wherein said imaging data are obtained by a non-invasive imaging method,
(e) mathematically combining, preferably in a binary logistic regression,

- the variables obtained in step (c), or any mathematical combination thereof with

- the data obtained at step (d),

wherein the mathematical combination results in a diagnostic score, and
(f) assessing the presence and/or severity of varices, selected from gastric and esophageal varices, preferably of large esophageal varices, based on the diagnostic score obtained in step (e).

In one embodiment, the imaging data on varices status are obtained by a non-invasive imaging method, preferably esophageal capsule endoscopy; or by a radiologic method, preferably a scanner.
In one embodiment, the non-invasive test carried out in step (a) is a blood test, preferably selected from ELF, FibroSpect™, APRI, FIB-4, Hepascore, Fibrotest™, FibroMeter™, CirrhoMeter™, CombiMeter, Elasto-FibroMeter™, Elasto-Fibrotest, InflaMeter™; or a physical method, preferably selected from VCTE, ARFI, VTE, supersonic elastometry or MRI stiffness.
In one embodiment, at step (c), the obtained variables are the variables of the non-invasive test carried out in step (a).
In one embodiment, the non-invasive test carried out in step (a) is a CirrhoMeter.
In one embodiment, the non-invasive test carried out in step (a) is a CirrhoMeter, and wherein the variables obtained at step (c) are the variables of a CirrhoMeter.

In one embodiment, the patient is affected with a chronic hepatic disease, preferably selected from the group comprising chronic viral hepatitis C, chronic viral hepatitis B, chronic viral hepatitis D, chronic viral hepatitis E, non-alcoholic fatty liver disease (NAFLD), alcoholic chronic liver disease, autoimmune hepatitis, primary biliary cirrhosis, hemochromatosis and Wilson disease.

In one embodiment, the patient is a cirrhotic patient.

Another object of the invention is a non-invasive method for assessing the presence and/or severity of varices, selected from gastric and esophageal varices, preferably of large esophageal varices, in a hepatic disease patient, wherein said method comprises:

i. measuring at least one of the following variables from the subject:

- biomarkers,
- clinical data,
- binary markers,
- physical data from medical imaging or clinical measurement

ii. obtaining imaging data on varices status, wherein said imaging data are obtained by a non-invasive imaging method,

iii. mathematically combining, preferably in a binary logistic regression,

- the variables obtained in step (i), or any mathematical combination thereof with

- the data obtained at step (ii),

wherein the mathematical combination results in a diagnostic score, and

iv. assessing the presence and/or severity of varices, selected from gastric and esophageal varices, preferably of large esophageal varices, based on the diagnostic score obtained in step (iii).

In one embodiment, the patient was previously diagnosed as cirrhotic, or wherein the patient previously obtained a value between the NPV and the PPV cut-offs in a method as described hereinabove.

The present invention also relates to a microprocessor comprising a computer algorithm carrying out the method as described hereinabove.

## DEFINITIONS

In the present invention, the following terms have the following meanings:

- "Positive predictive value (PPV)" refers to the proportion of patients with a positive test that actually have disease; if 9 of 10 positive test results are correct (true positive), the PPV is 90%. Because all positive test results have some number of true positives and some false positives, the PPV describes how likely it is that a positive test result in a given patient population represents a true positive.

- "Negative predictive value (NPV)" refers to the proportion of patients with a negative test result that are actually disease free; if 8 of 10 negative test results are correct (true negative), the NPV is 80%. Because not all negative test results are true negatives, some patients with a negative test result actually have disease. The NPV describes how likely it is that a negative test result in a given patient population represents a true negative.

- "AUROC" stands for area under the ROC curve, and is an indicator of the accuracy of a diagnostic test. In statistics, a receiver operating characteristic (ROC), or ROC curve, is a graphical plot that illustrates the performance of a binary classifier system as its discrimination threshold is varied. The curve is created by plotting the sensitivity against the specificity (usually 1- specificity) at successive values from 0 to 1. ROC curve and AUROC are well-known in the field of statistics.

- Sensitivity (also called true positive rate) measures the proportion of actual positives which are correctly identified as such.

- Specificity (also called true negative rate) measures the proportion of negatives which are correctly identified as such.

- "Esophageal varices" refers to dilated sub-mucosal veins in the lower third of the esophagus. Esophageal varices are a consequence of portal hypertension (referring to portal pressure of at least about 10 mm Hg, preferably at least about 12 mm Hg), commonly due to cirrhosis. As used herein, the term "large esophageal varices" may refer to varices of at least about 5 mm in diameter, such as, for example, when measured by UGEI. The term "large esophageal varices" may also refer to esophageal varices of at least 15 % of the esophageal circumference, preferably of at least 25, 30, 40, 50 % or more.

- "Gastric varices" refers to dilated sub-mucosal veins in the stomach. Gastric varices are a consequence of portal hypertension (referring to portal pressure of at least about 10 mm Hg, preferably at least about 12 mm Hg), commonly due to cirrhosis.

- "About" preceding a figure means plus or less 10% of the value of said figure.

- "Biomarker" refers to a variable that may be measured in a sample from the subject, wherein the sample may be a bodily fluid sample, such as, for example, a blood, serum or urine sample, preferably a blood or serum sample.

- "Clinical data" refers to a data recovered from external observation of the subject, without the use of laboratory tests and the like.

- "Binary marker" refers to a marker having the value 0 or 1 (or yes or no).

- "Physical data" refers to a variable obtained by a physical method.

- "Blood test" corresponds to a test comprising non-invasively measuring at least one data, and, when at least two data are measured, mathematically combining said at least two data within a score. In the present invention, said data may be a biomarker, a clinical data, a physical data, a binary marker or any combination thereof (such as, for example, any mathematical combination within a score).

- "Score" refers to any digit value obtained by the mathematical combination (univariate or multivariate) of at least one biomarker and/or at least one clinical data and/or at least one physical data and/or at least one binary marker and/or at least one blood test result. In one embodiment, a score is an unbound digit value. In another embodiment, a score is a bound digit value, obtained by a mathematical function. Preferably, a score ranges from 0 to 1. In one embodiment, the at least one biomarker and/or at least one clinical data and/or at least one physical data and/or at least one binary marker and/or at least one score, mathematically combined in a score are independent, i.e. give each an information that is different and not linked to the information given by the others.

- "Patient" refers to a subject awaiting the receipt of, or is receiving medical care or is/will be the object of a medical procedure for treating a hepatic disease.

## DETAILED DESCRIPTION

The present invention relates to non-invasive methods for assessing the presence and/or severity of varices, selected from esophageal varices and gastric varices in a liver disease patient, preferably is a patient with chronic liver disease.

In one embodiment, the method of the invention is an *in vitro* method.

In one embodiment, the method of the invention is for assessing the presence of esophageal varices, preferably of esophageal varices of at least about 1 mm in diameter, preferably of at least about 1.5, 2, 2.5, 3, 3.5, 4, 4.5, 5, 6, 7, 8, 9, 10, 15, or 20 mm or more, such as, for example, when measured by UGEI. In one embodiment, the method of the invention is for assessing the presence of large esophageal varices, i.e. of esophageal varices of at least 15 % of the esophageal circumference, preferably of at least 25, 30, 40, 50 % or more when measured by ECE, or varices of at least about 5 mm in diameter, such as, for example, when measured by UGEI.

In another embodiment, the method of the invention is for assessing the presence of gastric varices such as, for example, gastro-esophageal varices or preferably isolated gastric varices, usually fundal varices.

The present invention first relates to a non-invasive method for assessing the presence and/or severity of varices, selected from esophageal varices and gastric varices in a liver disease patient, using a non-invasive test for assessing the severity of a hepatic lesion or disorder.

However, in this usual test for assessing the severity of a hepatic lesion or disorder, the cut-offs of said test for assessing the presence and/or severity of varices are determined, preferably as described hereinabove.

Hence, this invention relates to a method comprising:

(a) carrying out the non-invasive test, wherein said non-invasive test results in a value, and
(b) comparing the value obtained at step (a) with said cut-offs of said test for assessing the presence and/or severity of varices.

In one embodiment, the method of the invention further comprise a first step of determining the cut-offs of said test for assessing the presence and/or severity of varices, using a population of reference.

Two cut-offs may usually be determined for diagnostic tests, i.e. the NPV cut-off and the PPV cut-off. A value below the NPV cut-off is indicative of the absence of the diagnostic target, whereas a value above the PPV cut-off is indicative of the presence of the diagnostic target. Between the NPV cut-off and the PPV cut-off is an indeterminate zone, wherein no conclusion may be raised regarding the presence or absence of the diagnosis target.

In one embodiment, the cut-offs are sensitivity cut-offs and specificity cut-offs. A value below the sensitivity cut-off is indicative of the absence of the diagnostic target, whereas a value above the specificity cut-off is indicative of the presence of the diagnostic target. Between the sensitivity cut-off and the specificity cut-off is an indeterminate zone, wherein no conclusion may be raised regarding the presence or absence of the diagnosis target.

In the present invention, the diagnostic target is the presence of varices, selected from gastric and esophageal varices (preferably large esophageal varices), and a value below the NPV cut-off is indicative of the absence of varices, selected from gastric and esophageal varices (preferably large esophageal varices), whereas a value above the PPV cut-off is indicative of the presence of varices, selected from gastric and esophageal varices (preferably large esophageal varices). Between the NPV cut-off and the PPV cut-off is an indeterminate zone, wherein no conclusion may be raised regarding the presence or absence of varices, selected from gastric or esophageal varices (preferably large esophageal varices).

In the present invention, the diagnostic target is the presence of varices, selected from gastric and esophageal varices (preferably large esophageal varices), and a value below the sensitivity cut-off is indicative of the absence of varices, selected from gastric and esophageal varices (preferably large esophageal varices), whereas a value above the specificity cut-off is indicative of the presence of varices, selected from gastric and esophageal varices (preferably large esophageal varices). Between the sensitivity cut-off and the specificity cut-off is an indeterminate zone, wherein no conclusion may be raised regarding the presence or absence of varices, selected from gastric and esophageal varices (preferably large esophageal varices).

The skilled artisan knows how to determine cut-offs for a diagnostic target (see for example a method for determining cut-offs of a diagnostic target in Cales, Liver Intern 2008), using a reference population.

In one embodiment, the NPV cut-offs and the PPV cut-offs are determined in a reference population in order to reach:

- a NPV of at least about 80%, preferably of at least about 85%, 86%, 87%, 88%, 89%, 90%, 91%, 92%, 93%, 94%, 95%, 96%, 97%, 98%, 99% or more, and/or
- a PPV of at least about 80%, preferably of at least about 85%, 86%, 87%, 88%, 89%, 90%, 91%, 92%, 93%, 94%, 95%, 96%, 97%, 98%, 99% or more.

In one embodiment, the NPV cut-offs and the PPV cut-offs are determined in a reference population in order to reach a NPV of at least 95% and a PPV of at least 90%.

In one embodiment, the sensitivity cut-offs and the specificity cut-offs are determined in a reference population in order to reach:

- a sensitivity of at least about 80%, preferably of at least about 85%, 86%, 87%, 88%, 89%, 90%, 91%, 92%, 93%, 94%, 95%, 96%, 97%, 98%, 99% or more, and/or
- a specificity of at least about 80%, preferably of at least about 85%, 86%, 87%, 88%, 89%, 90%, 91%, 92%, 93%, 94%, 95%, 96%, 97%, 98%, 99% or more.

In one embodiment, the sensitivity cut-offs and the specificity cut-offs are determined in a reference population in order to reach a sensitivity of at least 95% and a specificity of at least 90%.

In one embodiment, the reference population comprises liver disease patients, preferably patients with chronic liver disease, wherein for each patient the value of the non-invasive test was measured and the status regarding varices, selected from gastric and esophageal varices is known, i.e. absence or presence or size of varices, selected from gastric and esophageal varices, preferably of large esophageal varices (i.e. in one embodiment, an upper gastro-intestinal endoscopy was performed).

Therefore, the present invention is based on the application of a diagnostic test constructed for diagnosing the severity of a hepatic lesion or disorder to the diagnostic of another diagnostic target, varices, through the determination of cut-offs specific for esophageal varices diagnostic.

The experimental data provided in the Examples surprisingly demonstrated that the use of cut-offs specific for varices, selected from gastric and esophageal varices diagnostic instead of cut-offs specific for cirrhosis diagnosis increases the accuracy of the diagnostic test for diagnosing varices, selected from gastric and esophageal varices.

For example, CirrhoMeter™ is a non-invasive diagnostic test primarily constructed for diagnosing cirrhosis (i.e. cut-offs specific for cirrhosis were measured). In the present invention, CirrhoMeter™ cut-offs specific for esophageal varices (preferably large esophageal varices) were measured. CirrhoMeter™ cut-offs for cirrhosis or esophageal varices are shown in the table below.

| Diagnostic target | 95% NPV cut-off | 90% PPV cut-off |
|---|---|---|
| Cirrhosis | 0.302 | 0.725 |
| Esophageal varices | 0.545 | 0.9994 |

Therefore, in one embodiment, the method of the invention is for classifying a patient into one of the three following classes:

 i. absence of varices, selected from gastric and esophageal varices, preferably large esophageal varices (for patients having a value below the NPV cut-off value or below the sensitivity cut-off value)
 ii. presence of varices, selected from gastric and esophageal varices, preferably large esophageal varices (for patients having a value above the PPV cut-off value or above the specificity cut-off value) or
 iii. indeterminate zone (for patients having a value ranging between the NPV cut-off value and the PPV cut-off value or between the sensitivity cut-off value and the specificity cut-off value).

In one embodiment, the method of the invention further comprises, in particular for patients classified in the indeterminate zone, the following steps:

 (c) measuring at least one of the following variables from the subject:

 - biomarkers,
 - clinical data,
 - binary markers,
 - physical data from medical imaging or clinical measurement

 (d) obtaining imaging data on varices status, wherein said imaging data are obtained by a non-invasive imaging method,

 (e) mathematically combining:

 - the variables obtained in step (c), or any mathematical combination thereof with
 - the data obtained at step (d),

 wherein the mathematical combination results in a diagnostic score, and

 (f) assessing the presence and/or severity of varices, selected from gastric and esophageal varices (preferably large esophageal varices) based on the diagnostic score obtained in step (e).

In one embodiment, the assessment of the presence and/or severity of step (f) comprises comparing the score obtained in step (e) with cut-off values for the diagnostic test resulting in the diagnostic score of the invention. As explained hereinabove, two cut-offs may be determined for the diagnostic test resulting in the diagnostic score of the invention: the NPV cut-off and the PPV cut-off, or the sensitivity cut-off and the specificity cut-off.

In one embodiment, the NPV cut-offs and the PPV cut-offs are determined in a reference population in order to reach:

 - a NPV of at least about 75%, preferably of at least about 80%, more preferably of at least about 85%, 86%, 87%, 88%, 89%, 90%, 91%, 92%, 93%, 94%, 95%, 96%, 97%, 98%, 99% or more, and/or

- a PPV of at least about 75%, preferably of at least about 80%, preferably of at least about 85%, 86%, 87%, 88%, 89%, 90%, 91%, 92%, 93%, 94%, 95%, 96%, 97%, 98%, 99% or more.

In one embodiment, the NPV cut-offs and the PPV cut-offs are determined in a reference population in order to reach a NPV of at least 95% and a PPV of at least 90%.

In one embodiment, the sensitivity cut-offs and the specificity cut-offs are determined in a reference population in order to reach:

- a sensitivity of at least about 75%, preferably of at least about 80%, more preferably of at least about 85%, 86%, 87%, 88%, 89%, 90%, 91%, 92%, 93%, 94%, 95%, 96%, 97%, 98%, 99% or more, and/or
- a specificity of at least about 75%, preferably of at least about 80%, preferably of at least about 85%, 86%, 87%, 88%, 89%, 90%, 91%, 92%, 93%, 94%, 95%, 96%, 97%, 98%, 99% or more.

In one embodiment, the sensitivity cut-offs and the specificity cut-offs are determined in a reference population in order to reach a sensitivity of at least 95% and a PPV of at least 90%.

In the present invention, the diagnostic target is the presence of varices, selected from gastric and esophageal varices (preferably large esophageal varices), and a diagnostic score below the NPV (or sensitivity) cut-off is indicative of the absence of varices, selected from gastric and esophageal varices (preferably large esophageal varices), whereas a diagnostic score above the PPV (or specificity) cut-off is indicative of the presence of varices, selected from gastric and esophageal varices (preferably large esophageal varices). Between the NPV (or sensitivity) cut-off and the PPV (or specificity) cut-off is an indeterminate zone, wherein no conclusion may be raised regarding the presence or absence of varices, selected from gastric and esophageal varices (preferably large esophageal varices).

Therefore, in one embodiment, the method of the invention is for classifying a patient into one of the three following classes:

i. absence of varices, selected from gastric and esophageal varices, preferably absence of large esophageal varices (for patients having a diagnostic score below the NPV (or sensitivity) cut-off value)
ii. presence of varices, selected from gastric and esophageal varices, preferably presence of large esophageal varices (for patients having a diagnostic score above the PPV (or specificity) cut-off value) or
iii. indeterminate zone (for patients having a diagnostic score ranging between the NPV cut-off value and the PPV cut-off value or between the sensitivity and specificity cut-off values).

In one embodiment, patients having a diagnostic score between the NPV and PPV cut-offs required an invasive test for determining the presence or absence of varices, selected from gastric and esophageal varices, such as, for example, endoscopy (UGEI).

In one embodiment, patients having a diagnostic score between the sensitivity and specificity cut-offs required an invasive test for determining the presence or absence of varices, selected from gastric and esophageal varices, such as, for example, endoscopy (UGEI).

In one embodiment, in step (c), the obtained variables are the variables of the non-invasive test carried out in step (a).

In one embodiment, at step (e), the variables obtained at step (c) are mathematically combined in a non-invasive test value, preferably in a score, prior to the mathematical combination with the data obtained at step (d).

In one embodiment, the present invention thus relates to a non-invasive method for assessing the presence and/or severity of varices, selected from gastric and esophageal varices (preferably of large esophageal varices) in a liver disease patient, preferably in a patient with chronic liver disease, wherein said method comprises:

(a) carrying out a non-invasive test for assessing the severity of a hepatic lesion or disorder, wherein said non-invasive test results in a value, and

(b) comparing the value obtained at step (a) with cut-offs of said non-invasive test for assessing the presence and/or severity of varices, selected from gastric and esophageal varices (preferably large esophageal varices), thereby determining if the patient does not present varices, selected from gastric and esophageal varices, presents varices, selected from gastric and esophageal varices or is in an indeterminate zone, and
for patients in the indeterminate zone, the method of the invention further comprises:

(c) measuring at least one of the following variables from the subject:

- biomarkers,

- clinical data,
- binary markers,
- physical data from medical imaging or clinical measurement

(d) obtaining imaging data on varices status, wherein said imaging data are obtained by a non-invasive imaging method,

(e) mathematically combining

- the variables obtained in step (c), or any mathematical combination thereof with
- the data obtained at step (d),

wherein the mathematical combination results in a diagnostic score, and

(f) assessing the presence and/or severity of varices, selected from gastric and esophageal varices (preferably large esophageal varices) based on the diagnostic score obtained in step (e).

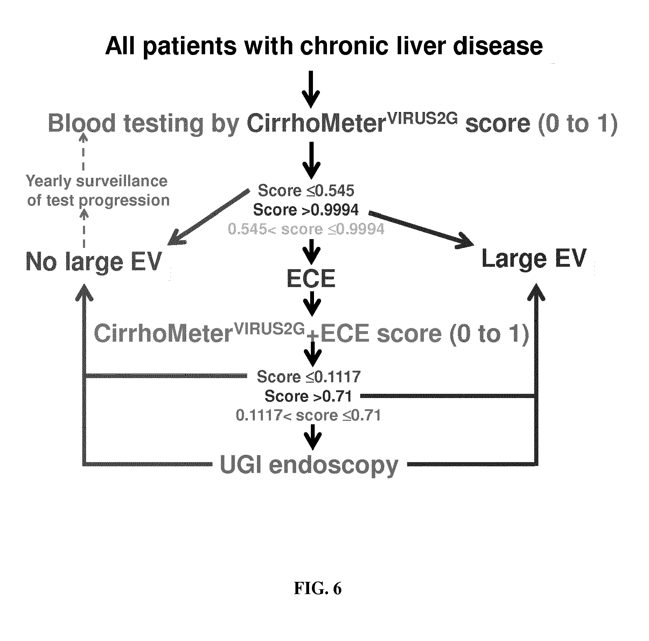

An algorithm corresponding to the non-invasive diagnostic method of the invention is shown in Figure 6.

In one embodiment, the determination of cut-offs for gastric varices is performed in the same way as for large esophageal varices (as illustrated in the Examples): first those of non-invasive test and then those of a score combining non-invasive test and ECE.

In one embodiment, the non-invasive test for assessing the severity of a hepatic lesion or disorder is a biomarker, a clinical data, a binary marker, a blood test or a physical method.

In one embodiment, the non-invasive test results in a value, preferably in a score.

In one embodiment, the non-invasive test is selected from the group comprising age, spleen diameter, ALT, leucocytes, body mass index, GGT, alpha2-macroglobulin, weight, segmented leucocytes, height, monocytes, hemoglobin, P2/MS score, alpha-fetoprotein, alkaline phosphatases, sodium, platelets, AST, InflaMeter, creatinine, urea, APRI, Child-Pugh score, FIB-4, VCTE, albumin, FibroMeter (such as, for example, FibroMeter for cause, FibroMeter$^{V2G}$ or FibroMeter$^{V3G}$), prothrombin index, CirrhoMeter (such as, for example, CirrhoMeter$^{V2G}$ or CirrhoMeter$^{V3G}$), bilirubin, Elasto-FibroMeter (such as, for example, Elasto-FibroMeter$^{V2G}$), hyaluronate, QuantiMeter (such as, for example, QuantiMeter for cause or QuantiMeter$^{V2G}$), Hepascore, Fibrotest, Fibrospect, Elasto-Fibrotest, ELF score and any mathematical combination thereof, such as, for example, AST/ALT, AST/ALT+prothrombin, AST/ALT+hyaluronate.

Examples of biomarkers include, but are not limited to, glycemia, total cholesterol, HDL cholesterol (HDL), LDL cholesterol (LDL), AST (aspartate aminotransferase), ALT (alanine aminotransferase), ferritin, platelets (PLT), prothrombin time (PT) or prothrombin index (PI) or INR (International Normalized Ratio), hyaluronic acid (HA or hyaluronate), haemoglobin, triglycerides, alpha-2 macroglobulin (A2M), gamma-glutamyl transpeptidase (GGT), urea, bilirubin (such as, for example, total bilirubin), apolipoprotein A1 (ApoA1), type III procollagen N-terminal propeptide (P3NP or P3P), gamma-globulins (GBL), sodium (Na), albumin (ALB) (such as, for example, serum albumin), ferritine (Fer), glucose (Glu), alkaline phosphatases (ALP), YKL-40 (human cartilage glycoprotein 39), tissue inhibitor of matrix metalloproteinase 1 (TIMP-1), TGF, cytokeratine 18 and matrix metalloproteinase 2 (MMP-2) to 9 (MMP-9), haptoglobin, alpha-fetoprotein, creatinine, leukocytes, neutrophils, segmented leukocytes, segmented neutrophils, monocytes, ratios and mathematical combinations thereof, such as, for example AST/ALT (ratio), AST.ALT (product), AST/PLT (ratio), AST/ALT+prothrombin, AST/ALT+hyaluronate.

The measurements carried out in the method of the invention are measurements aimed either at quantifying the biomarker (such as, for example, in the case of A2M, HA, bilirubin, PLT, PT, urea, NA, glycemia, triglycerides, ALB or P3P), or at quantifying the enzymatic activity of the biomarker (such as, for example, in the case of GGT, ASAT, ALAT, ALP). Those skilled in the art are aware of various direct or indirect methods for quantifying a given substance or a protein or its enzymatic activity. These methods may use one or more monoclonal or polyclonal antibodies that recognize said protein in immunoassay techniques (such as, for example, radioimmunoassay or RIA, ELISA assays, Western blot, etc.), the analysis of the amounts of mRNA for said protein using techniques of the Northern blot, slot blot or PCR type, techniques such as an HPLC optionally combined with mass spectrometry, etc. The abovementioned protein activity assays use assays carried out on at least one substrate specific for each of these proteins. International patent application WO 03/073822 lists methods that can be used to quantify alpha2 macroglobulin (A2M) and hyaluronic acid (HA or hyaluronate).

By way of examples, and in a non-exhaustive manner, a preferred list of commercial kits or assays that can be used for the measurements of biomarkers carried out in the method of the invention, on blood samples, is given hereinafter:

- prothrombin time: the Quick time (QT) is determined by adding calcium thromboplastin (for example, Neoplastin CI plus, Diagnostica Stago, Asnieres, France) to the plasma and the clotting time is measured in seconds. To obtain the prothrombin time (PT), a calibration straight line is plotted from various dilutions of a pool of normal plasmas estimated at 100%. The results obtained for the plasmas of patients are expressed as a percentage relative to the pool of normal plasmas. The upper value of the PT is not limited and may exceed 100%.
- A2M: the assaying thereof is carried out by laser immunonephelometry using, for example, a Behring nephelometer analyzer. The reagent may be a rabbit antiserum against human A2M.
- HA: the serum concentrations are determined with an ELISA (for example: Corgenix, Inc. Biogenic SA 34130 Mauguio France) that uses specific HA-binding proteins isolated from bovine cartilage.
- P3P: the serum concentrations are determined with an RIA (for example: RIA-gnost PIIIP kit, Hoechst, Tokyo, Japan) using a murine monoclonal antibody directed against bovine skin PIIINP.
- PLT: blood samples are collected in vacutainers containing EDTA (ethylenediaminetetraacetic acid) (for example, Becton Dickinson, France) and can be analyzed on an Advia 120 counter (Bayer Diagnostic).
- Urea: assaying, for example, by means of a "Kinectic UV assay for urea" (Roche Diagnostics).
- GGT: assaying, for example, by means of a "gamma-glutamyltransferase assay standardized against Szasz" (Roche Diagnostics).
- Bilirubin: assaying, for example, by means of a "Bilirubin assay" (Jendrassik-Grof method) (Roche Diagnostics).
- ALP: assaying, for example, by means of "ALP IFCC" (Roche Diagnostics).
- ALT: assaying, for example, by "ALT IFCC" (Roche Diagnostics).
- AST: assaying, for example, by means of "AST IFCC" (Roche Diagnostics). Sodium: assaying, for example, by means of "Sodium ion selective electrode" (Roche Diagnostics).
- Glycemia: assaying, for example, by means of "glucose GOD-PAP" (Roche Diagnostics).
- Triglycerides: assaying, for example, by means of "triglycerides GPO-PAP" (Roche Diagnostics).
- Urea, GGT, bilirubin, alkaline phosphatases, sodium, glycemia, ALT and AST can be assayed on an analyzer, for example, a Hitachi 917, Roche Diagnostics GmbH, D-68298 Mannheim, Germany.
- Gamma-globulins, albumin and alpha-2 globulins: assaying on protein electrophoresis, for example: capillary electrophoresis (Capillarys), SEBIA 23, rue M Robespierre, 92130 Issy Les Moulineaux, France.
- ApoA1: assaying, for example, by means of "Determination of apolipoprotein A-1" (Dade Behring) with an analyzer, for example: BN2 Dade Behring Marburg GmbH, Emil von Behring Str. 76, D-35041 Marburg, Germany.
- TIMP1: assaying, for example, by means of TIMP1-ELISA, Amersham.
- MMP2: assaying, for example, by means of MMP2-ELISA, Amersham.
- YKL-40: assaying, for example, by means of YKL-40 Biometra, YKL-40/8020, Quidel Corporation.
- PIIIP: assaying, for example, by means of PIIIP RIA kit, OCFKO7-PIIIP, cis bio international.

For the biomarkers measured in the method of the present invention, the values obtained may be expressed in:

- mg/dl, such as, for example, for alpha2-macroglobulin (A2M),
- $\mu$g/l, such as, for example, for hyaluronic acid (HA or hyaluronate), or ferritin
- g/l, such as, for example, for apolipoprotein A1 (ApoA1), gamma-globulins (GLB) or albumin (ALB)
- U/ml, such as, for example, for type III procollagen N-terminal propeptide (P3P)
- IU/l, such as, for example, for gamma-glutamyltranspeptidase (GGT), aspartate aminotransferases (AST), alanine aminotransferases (ALT) or alkaline phosphatases (ALP)
- $\mu$mol/1, such as, for example, for bilirubin,
- Giga/l, such as, for example, for platelets (PLT),
- %, such as, for example, for prothrombin time (PT),
- mmol/l, such as, for example, for triglycerides, urea, sodium (NA), glycemia, or
- ng/ml, such as, for example, for TIMP1, MMP2, or YKL-40.

Examples of clinical data include, but are not limited to, weight, height, body mass index, age, sex, hip perimeter, abdominal perimeter or height, spleen diameter (preferably by abdominal imaging), and mathematical combinations thereof, such as, for example, the ratio thereof, such as for example hip perimeter/abdominal perimeter.
Examples of non-invasive binary markers include, but are not limited to, diabetes, SVR (wherein SVR stands for sustained virologic response, and is defined as aviremia 6 weeks, preferably 12 weeks, more preferably 24 weeks after completion of antiviral therapy for chronic hepatitis C virus (HCV) infection), etiology, hepatic encephalopathy, ascites, and NAFLD. Regarding the binary marker "etiology", the skilled artisan knows that said variable is a single or multiple binary marker, and that for liver disorders, etiology may be NAFLD, alcohol, virus or other. Thus, the binary marker might be expressed as NAFLD vs others (single binary marker) or as NAFLD vs reference etiology plus virus vs reference etiology and so on (multiple binary marker).

Preferably, the data is an elastometry data, preferably Liver Stiffness Evaluation (LSE) data or spleen stiffness evaluation, which may be for example obtained by VCTE or ARFI or SSI or another elsatometric technique. According to a preferred embodiment of the invention, the physical data is liver stiffness measurement (LSM), preferably measured by VCTE.

In a particular embodiment, the physical data is Liver stiffness measurement (LSM) by VCTE (also known as Fibroscan™, Paris, France), preferably performed with the M probe. Preferably, examination conditions are those recommended by the manufacturer, with the objective of obtaining at least 3 and preferably 10 valid measurements. Results may be expressed as the median (kilopascals) of all valid measurements, or as IQR or as the ratio (IQR/median).

In one embodiment, the blood test of the invention corresponds to a blood test selected from the group comprising ELF, FibroSpect™, APRI, FIB-4, Hepascore, Fibrotest™, FibroMeter™ (such as, for example, FibroMeter for cause, FibroMeter$^{V2G}$ or FibroMeter$^{V3G}$), CirrhoMeter™ (such as, for example, CirrhoMeter$^{V2G}$ or CirrhoMeter$^{V3G}$), CombiMeter, Elasto-FibroMeter™ (such as, for example, Elasto-FibroMeter$^{V2G}$), InflaMeter™, Actitest, QuantiMeter, P2/MS score, Elasto-Fibrotest, and Child-Pugh score. As these blood tests are diagnostic tests, they can be based on multivariate mathematical combination, such as, for example, binary logistic regression, or include clinical data.

ELF is a blood test based on hyaluronic acid, P3P, TIMP-1 and age.

FibroSpect™ is a blood test based on hyaluronic acid, TIMP-1 and A2M.

APRI is a blood test based on platelet and AST.

FIB-4 is a blood test based on platelet, AST, ALT and age.

HEPASCORE is a blood test based on hyaluronic acid, bilirubin, alpha2-macroglobulin, GGT, age and sex.

FIBROTEST™ is a blood test based on alpha2-macroglobulin, haptoglobin, apolipoprotein A1, total bilirubin, GGT, age and sex.

FIBROMETER™ and CIRRHOMETER™ together form a family of blood tests, the content of which depends on the cause of chronic liver disease and the diagnostic target (such as, for example, fibrosis, significant fibrosis or cirrhosis). This blood test family is called FM family and is detailed in the table below.

| Cause | Variables | | | | | | | | | | | | |
|---|---|---|---|---|---|---|---|---|---|---|---|---|---|
| | Age | Sex | Weigth | A2M | HA | PI | PLT | AST | Urea | GGT | ALT | Fer | Glu |
| **Virus** | | | | | | | | | | | | | |
| FM V 1G | x | | | x | x | x | x | x | x | | | | |
| FM V 2G | x | x | | x | x | x | x | x | x | | | | |
| CM V 2G | x | x | | x | x | x | x | x | x | | | | |
| FM V 3G[a] | x | x | | x | | x | x | x | x | x | | | |
| CM V 3G[a] | x | x | | x | | x | x | x | x | x | | | |
| **Alcohol** | | | | | | | | | | | | | |
| FM A 1G | x | | | x | x | x | | | | | | | |
| FM A 2G | | | | x | x | x | | | | | | | |
| **NAFLD** (steatosis) | | | | | | | | | | | | | |
| FM S | x | | x | | | | x | x | | | x | x | x |
| FM: FibroMeter, CM: CirrhoMeter, <br> A2M: alpha-2 macroglobulin, HA: hyaluronic acid, PI: prothrombin index, PLT: platelets, Fer: ferritin, Glu: glucose <br> [a] HA is replaced by GGT | | | | | | | | | | | | | |

COMBIMETER™ or Elasto-FibroMeter™ is a family of tests based on the mathematical combination of variables of the FM family (as detailed in the Table hereinabove) or of the result of a test of the FM family with VCTE (FIBROSCAN™) result. In one embodiment, said mathematical combination is a binary logistic regression.

In one embodiment, CombiMeter™ or Elasto-FibroMeter™ results in a score based on the mathematical combination of physical data from liver or spleen elastometry such as dispersion index from VCTE (Fibroscan™) such as IQR or IQR/median or median of LSM, preferably of LSM (by Fibroscan™) median with at least 3, 4, 5, 6, 7, 8 or 9 biomarkers and/or clinical data selected from the list comprising glycemia, total cholesterol, HDL cholesterol (HDL), LDL cholesterol (LDL), AST (aspartate aminotransferase), ALT (alanine aminotransferase), AST/ALT, AST.ALT, ferritin, platelets (PLT), AST/PLT, prothrombin time (PT) or prothrombin index (PI), hyaluronic acid (HA or hyaluronate), haemoglobin, triglycerides, alpha-2 macroglobulin (A2M), gamma-glutamyl transpeptidase (GGT), urea, bilirubin, apolipoprotein A1 (ApoA1), type III procollagen N-terminal propeptide (P3NP), gamma-globulins (GBL), sodium (Na), albumin (ALB), ferritine (Fer), glucose (Glu), alkaline phosphatases (ALP), YKL-40 (human cartilage glycoprotein 39), tissue inhibitor of matrix metalloproteinase 1 (TIMP-1), TGF, cytokeratine 18 and matrix metalloproteinase 2 (MMP-2) to 9 (MMP-9), haptoglobin, diabetes, weight, body mass index, age, sex, hip perimeter, abdominal perimeter or height and ratios and mathematical combinations thereof.

INFLAMETER™ is a companion test reflecting necro-inflammatory activity including ALT, A2M, PI, and platelets.

ACTITEST is a blood test based on alpha2-macroglobulin, haptoglobin, apolipoprotein A1, total bilirubin, GGT, ALT, age and sex.

QUANTIMETER is a blood test based on (i) alpha2-macroglobulin, hyaluronic acid, prothrombin time, platelets when designed for alcoholic liver diseases, (ii) hyaluronic acid, prothrombin time, platelets, AST, ALT and glycemia when designed for NAFLD, or (iii) alpha2-macroglobulin, hyaluronic acid, platelets, urea, GGT and bilirubin when designed for chronic viral hepatitis.

P2/MS is a blood test based on platelet count, monocyte fraction and segmented neutrophil fraction.

CHILD-PUGH SCORE is a blood test based on total bilirubin, serum albumin, PT or INR, ascites and hepatic encephalopathy.

ELASTO-FIBROTEST is a test based on the mathematical combination of variables of FIBROTEST or of the result of a FIBROTEST, with LSM measurement, measured for example by Fibroscan™.

In one embodiment, step (a) of the non-invasive method of the invention comprises measuring and combining in a mathematical function the variables of ELF, i.e. hyaluronic acid, P3P, TIMP-1 and age.

In one embodiment, step (a) of the non-invasive method of the invention comprises measuring and combining in a mathematical function the variables of FibroSpect™, i.e. hyaluronic acid, TIMP-1 and A2M.

In one embodiment, step (a) of the non-invasive method of the invention comprises measuring and combining in a mathematical function the variables of APRI, i.e. platelet and AST.

In one embodiment, step (a) of the non-invasive method of the invention comprises measuring and combining in a mathematical function the variables of FIB-4, i.e. platelet, AST, ALT and age.

In one embodiment, step (a) of the non-invasive method of the invention comprises measuring and combining in a mathematical function the variables of HEPASCORE, i.e. hyaluronic acid, bilirubin, alpha2-macroglobulin, GGT, age and sex.

In one embodiment, step (a) of the non-invasive method of the invention comprises measuring and combining in a mathematical function the variables of FIBROTEST™, i.e. alpha2-macroglobulin, haptoglobin, apolipoprotein A1, total bilirubin, GGT, age and sex.

In one embodiment, step (a) of the non-invasive method of the invention comprises measuring and combining in a mathematical function the variables of FIBROMETER™ and/or CIRRHOMETER™ as defined hereinabove.

In one embodiment, step (a) of the non-invasive method of the invention comprises measuring and combining in a mathematical function the variables of CIRRHOMETER™, i.e. the following variables:

- age, sex, alpha2-macroglobulin, hyaluronic acid, prothrombin time, platelets, AST and urea, or
- age, sex, alpha2-macroglobulin, gamma-glutamyl transpeptidase, prothrombin time, platelets, AST and urea.

In one embodiment, step (a) of the non-invasive method of the invention comprises measuring and combining in a mathematical function the variables of COMBIMETER™ or Elasto-FibroMeter™ as defined hereinabove.

In one embodiment, step (a) of the non-invasive method of the invention comprises measuring and combining in a mathematical function the variables of INFLAMETER™, i.e. ALT, A2M, PI, and platelets.

In one embodiment, step (a) of the non-invasive method of the invention comprises measuring and combining in a mathematical function the variables of ACTITEST, i.e. alpha2-macroglobulin, haptoglobin, apolipoprotein A1, total bilirubin, GGT, ALT, age and sex.

In one embodiment, step (a) of the non-invasive method of the invention comprises measuring and combining in a mathematical function the variables of QUANTIMETER, i.e. (i) alpha2-macroglobulin, hyaluronic acid, prothrombin time, platelets, (ii) hyaluronic acid, prothrombin time, platelets, AST, ALT and glycemia, or (iii) alpha2-macroglobulin, hyaluronic acid, platelets, urea, GGT and bilirubin.

In one embodiment, step (a) of the non-invasive method of the invention comprises measuring and combining in a mathematical function the variables of P2/MS score, i.e. platelet count, monocyte fraction and segmented neutrophil fraction.

In one embodiment, step (a) of the non-invasive method of the invention comprises measuring and combining in a mathematical function the variables of CHILD-PUGH SCORE, i.e. total bilirubin, serum albumin, PT or INR, ascites and hepatic encephalopathy.

In one embodiment, step (c) of the non-invasive method of the invention comprises measuring the variables of ELF, i.e. hyaluronic acid, P3P, TIMP-1 and age.

In one embodiment, step (c) of the non-invasive method of the invention comprises measuring the variables of FibroSpect™, i.e. hyaluronic acid, TIMP-1 and A2M.

In one embodiment, step (c) of the non-invasive method of the invention comprises measuring the variables of APRI, i.e. platelet and AST.

In one embodiment, step (c) of the non-invasive method of the invention comprises measuring the variables of FIB-4, i.e. platelet, AST, ALT and age.

In one embodiment, step (c) of the non-invasive method of the invention comprises measuring the variables of HEPASCORE, i.e. hyaluronic acid, bilirubin, alpha2-macroglobulin, GGT, age and sex.

In one embodiment, step (c) of the non-invasive method of the invention comprises measuring the variables of FIBROTEST™, i.e. alpha2-macroglobulin, haptoglobin, apolipoprotein A1, total bilirubin, GGT, age and sex.

In one embodiment, step (c) of the non-invasive method of the invention comprises measuring the variables of FIBROMETER™ and/or CIRRHOMETER™ as defined hereinabove.

In one embodiment, step (c) of the non-invasive method of the invention comprises measuring the variables of CIRRHOMETER™, i.e. the following variables:

- age, sex, alpha2-macroglobulin, hyaluronic acid, prothrombin time, platelets, AST and urea, or
- age, sex, alpha2-macroglobulin, gamma-glutamyl transpeptidase, prothrombin time, platelets, AST and urea.

In one embodiment, step (c) of the non-invasive method of the invention comprises measuring the variables of COMBIMETER™ or Elasto-FibroMeter™ as defined hereinabove.

In one embodiment, step (c) of the non-invasive method of the invention comprises measuring the variables of INFLAMETER™, i.e. ALT, A2M, PI, and platelets.

In one embodiment, step (c) of the non-invasive method of the invention comprises measuring the variables of ACTITEST, i.e. alpha2-macroglobulin, haptoglobin, apolipoprotein A1, total bilirubin, GGT, ALT, age and sex.

In one embodiment, step (c) of the non-invasive method of the invention comprises measuring the variables of QUANTIMETER, i.e. (i) alpha2-macroglobulin, hyaluronic acid, prothrombin time, platelets, (ii) hyaluronic acid, prothrombin time, platelets, AST, ALT and glycemia, or (iii) alpha2-macroglobulin, hyaluronic acid, platelets, urea, GGT and bilirubin.

In one embodiment, step (c) of the non-invasive method of the invention comprises measuring the variables of P2/MS score, i.e. platelet count, monocyte fraction and segmented neutrophil fraction.

In one embodiment, step (c) of the non-invasive method of the invention comprises measuring the variables of CHILD-PUGH SCORE, i.e. total bilirubin, serum albumin, PT or INR, ascites and hepatic encephalopathy.

Examples of physical methods include, but are not limited to, medical imaging data and clinical measurements, such as, for example, measurement of spleen, especially spleen length (that may also be referred as diameter). According to an embodiment, the physical method is selected from the group comprising ultrasonography, especially Doppler-ultrasonography and elastometry ultrasonography and velocimetry ultrasonography (preferred tests using said data are vibration controlled transient elastography (VCTE, also known as Fibroscan™), ARFI, VTE, supersonic elastometry (supersonic imaging), MRI (Magnetic Resonance Imaging), and MNR (Magnetic Nuclear Resonance) as used in spectroscopy, especially MNR elastometry or velocimetry.

In one embodiment, the physical method is VCTE, ARFI, VTE, supersonic elastometry or MRI stiffness.

In one embodiment of the invention, the method of the invention comprises carrying out a VCTE, which refers to obtaining at least 3 and preferably 10 valid measurements and recovering a physical data corresponding to the median in kilopascals of all valid measurements.

In one embodiment, the present invention non-invasive method for assessing the presence and/or severity of esophageal varices in a hepatic disease patient comprises:

(a) carrying out a CirrhoMeter (such as, for example, a CirrhoMeter[V2G] or a CirrhoMeter[V3G], preferably a CirrhoMeter[V2G]), resulting in a CirrhoMeter score, and

(b) comparing the CirrhoMeter score obtained at step (a) with cut-offs of said CirrhoMeter for assessing the presence and/or severity of esophageal varices, thereby determining if the patient does not present esophageal varices (preferably large esophageal varices), presents esophageal varices or is in an indeterminate zone, and
for patients in the indeterminate zone, the method of the invention further comprises:

(c) measuring the variables of CirrhoMeter in the subject,

(d) obtaining imaging data on varices status, wherein said imaging data are obtained by a non-invasive imaging method,

(e) mathematically combining

- the variables obtained in step (c), or any mathematical combination thereof in a CirrhoMeter (such as, for example, a CirrhoMeter$^{V2G}$ or a CirrhoMeter$^{V3G}$, preferably a CirrhoMeter$^{V2G}$) with
- the data obtained at step (d),

wherein the mathematical combination results in a diagnostic score, and

(f) assessing the presence and/or severity of esophageal varices based on the diagnostic score obtained in step (e).

In one embodiment, the present invention non-invasive method for assessing the presence and/or severity of varices, selected from gastric and esophageal varices in a hepatic disease patient comprises:

(a) obtaining and mathematically combining in a CirrhoMeter (such as, for example, a CirrhoMeter$^{V2G}$ or a CirrhoMeter$^{V3G}$, preferably a CirrhoMeter$^{V2G}$), the following variables:

- age, sex, alpha2-macroglobulin, hyaluronic acid, prothrombin time, platelets, AST and urea, or
- age, sex, alpha2-macroglobulin, gamma-glutamyl transpeptidase, prothrombin time, platelets, AST and urea

thereby obtaining a CirrhoMeter score, and

(b) comparing the CirrhoMeter score obtained at step (a) with cut-offs of said CirrhoMeter for assessing the presence and/or severity of esophageal varices, thereby determining if the patient does not present esophageal varices, presents esophageal varices (preferably large esophageal varices) or is in an indeterminate zone, and
for patients in the indeterminate zone, the method of the invention further comprises:

(c) measuring the following variables in the subject:

- age, sex, alpha2-macroglobulin, hyaluronic acid, prothrombin time, platelets, AST and urea, or
- age, sex, alpha2-macroglobulin, gamma-glutamyl transpeptidase, prothrombin time, platelets, AST and urea,

(d) obtaining imaging data on varices status, wherein said imaging data are obtained by a non-invasive imaging method,

(e) mathematically combining

- the variables obtained in step (c), or any mathematical combination thereof in a CirrhoMeter (such as, for example, a CirrhoMeter$^{V2G}$ or a CirrhoMeter$^{V3G}$, preferably a CirrhoMeter$^{V2G}$) with
- the data obtained at step (d),

wherein the mathematical combination results in a diagnostic score, and

(f) assessing the presence and/or severity of varices selected from gastric and esophageal varices, based on the diagnostic score obtained in step (e).

Examples of non-invasive imaging data allowing the assessment of varices status (i.e. for visualizing varices or the absence of varices) include data obtained with non-invasive imaging methods or radiology.
Examples of non-invasive imaging methods for assessing varices status include, but are not limited to, esophageal capsule endoscopy (ECE), CT-scan, echo-endoscopy or MRI. Examples of esophageal capsules that may be used in the method of the present invention includes esophageal capsules developed by Given-covidien-medtronic.
Examples of radiologic methods for assessing varices status include, but are not limited to, CT-scanner and MRI.
In one embodiment, the non-invasive imaging data corresponds to a grade according to the size of the visualized varices:

- grade 0: absence of varices,
- grade 1: presence of small varices of less than 5 mm in diameter or 15 to 25% of esophageal circumference, and

- grade 2: presence of large varices (i.e. of at least about 5 mm in diameter or 15 to 25% of esophageal circumference).

In one embodiment, the step (a) of the method of the invention comprises carrying out a CirrhoMeter, such as, for example, a CirrhoMeter$^{V2G}$ or a CirrhoMeter$^{V3G}$, preferably a CirrhoMeter$^{V2G}$.

In one embodiment, the step (c) of the method of the invention comprises carrying out a CirrhoMeter, such as, for example, a CirrhoMeter$^{V2G}$ or a CirrhoMeter$^{V3G}$, preferably a CirrhoMeter$^{V2G}$.

In one embodiment, the step (a) and step (c) of the method of the invention both comprise carrying out a CirrhoMeter, such as, for example, a CirrhoMeter$^{V2G}$ or a CirrhoMeter$^{V3G}$, preferably a CirrhoMeter$^{V2G}$.

In one embodiment, the step (a) of the method of the invention comprises carrying out a FibroMeter, such as, for example, a FibroMeter$^{V2G}$ or a FibroMeter$^{V3G}$.

In one embodiment, the step (c) of the method of the invention comprises carrying out a FibroMeter, such as, for example, a FibroMeter$^{V2G}$ or a FibroMeter$^{V3G}$.

In one embodiment, the step (a) and step (c) of the method of the invention both comprise carrying out a FibroMeter, such as, for example, a FibroMeter$^{V2G}$ or a FibroMeter$^{V3G}$.

In one embodiment, the step (d) of the method of the invention comprises obtaining imaging data obtained by ECE.

In one embodiment, the step (e) of the method of the invention comprises mathematically combining a CirrhoMeter (such as, for example, a CirrhoMeter$^{V2G}$ or a CinhoMeter$^{V3G}$, preferably a CirrhoMeter$^{V2G}$) or the variables of a CirrhoMeter (such as, for example, a CirrhoMeter$^{V2G}$ or a CirrhoMeter$^{V3G}$, preferably a CirrhoMeter$^{V2G}$) with a data obtained by ECE.

In one embodiment, the step (e) of the method of the invention comprises mathematically combining a FibroMeter (such as, for example, a FibroMeter$^{V2G}$ or a FibroMeter$^{V3G}$,) or the variables of a FibroMeter (such as, for example, a FibroMeter$^{V2G}$ or a FibroMeter$^{V3G}$) with a data obtained by ECE.

In one embodiment, the step (c) of the method of the invention comprises carrying out a CirrhoMeter, such as, for example, a CirrhoMeter$^{V2G}$ or a CirrhoMeter$^{V3G}$, preferably a CirrhoMeter$^{V2G}$; the step (d) of the method of the invention comprises obtaining imaging data obtained by ECE; and the step (e) of the method of the invention comprises mathematically combining the result of the CirrhoMeter carried out at step (c) with the data obtained by ECE.

In one embodiment, the step (c) of the method of the invention comprises carrying out a FibroMeter, such as, for example, a FibroMeter$^{V2G}$ or a FibroMeter$^{V3G}$; the step (d) of the method of the invention comprises obtaining imaging data obtained by ECE; and the step (e) of the method of the invention comprises mathematically combining the result of the FibroMeter carried out at step (c) with the data obtained by ECE.

In one embodiment, the step (a) of the method of the invention comprises carrying out a CirrhoMeter, such as, for example, a CirrhoMeter$^{V2G}$ or a CirrhoMeter$^{V3G}$, preferably a CirrhoMeter$^{V2G}$; the step (c) of the method of the invention comprises carrying out a CirrhoMeter, such as, for example, a CirrhoMeter$^{V2G}$ or a CirrhoMeter$^{V3G}$, preferably a CirrhoMeter$^{V2G}$; the step (d) of the method of the invention comprises obtaining imaging data obtained by ECE; and the step (e) of the method of the invention comprises mathematically combining the result of the CirrhoMeter carried out at step (c) with the data obtained by ECE.

In one embodiment, the step (a) of the method of the invention comprises carrying out a FibroMeter, such as, for example, a FibroMeter$^{V2G}$ or a FibroMeter$^{V3G}$; the step (c) of the method of the invention comprises carrying out a FibroMeter, such as, for example, a FibroMeter$^{V2G}$ or a FibroMeter$^{V3G}$; the step (d) of the method of the invention comprises obtaining imaging data obtained by ECE; and the step (e) of the method of the invention comprises mathematically combining the result of the FibroMeter carried out at step (c) with the data obtained by ECE.

In one embodiment, the step (a) of the method of the invention comprises carrying out a FibroMeter, such as, for example, a FibroMeter$^{V2G}$ or a FibroMeter$^{V3G}$; the step (c) of the method of the invention comprises carrying out a CirrhoMeter, such as, for example, a CirrhoMeter$^{V2G}$ or a CirrhoMeter$^{V3G}$, preferably a CirrhoMeter$^{V2G}$; the step (d) of the method of the invention comprises obtaining imaging data obtained by ECE; and the step (e) of the method of the invention comprises mathematically combining the result of the CirrhoMeter carried out at step (c) with the data obtained by ECE.

In one embodiment, the step (a) of the method of the invention comprises carrying out a CirrhoMeter, such as, for example, a CirrhoMeter$^{V2G}$ or a CirrhoMeter$^{V3G}$, preferably a CirrhoMeter$^{V2G}$; the step (c) of the method of the invention comprises carrying out a FibroMeter, such as, for example, a FibroMeter$^{V2G}$ or a FibroMeter$^{V3G}$; the step (d) of the method of the invention comprises obtaining imaging data obtained by ECE; and the step (e) of the method of the invention comprises mathematically combining the result of the FibroMeter carried out at step (c) with the data obtained by ECE.

In one embodiment, the patient is a mammal, preferably a human. In one embodiment, the patient is a male or a female. In one embodiment, the patient is an adult or a child.

In one embodiment, the patient is affected, preferably is diagnosed with a liver disease or disorder.

In one embodiment, the patient is affected with a liver disease or disorder, preferably selected from the list comprising significant porto-septal fibrosis, severe porto-septal fibrosis, centrolobular fibrosis, cirrhosis, persinusoidal fibrosis, the fibrosis being from alcoholic or non-alcoholic origin.

In one embodiment, the patient is affected with a chronic disease, preferably said chronic disease is selected from the group comprising chronic viral hepatitis C, chronic viral hepatitis B, chronic viral hepatitis D, chronic viral hepatitis E, non-alcoholic fatty liver disease (NAFLD), alcoholic chronic liver disease, autoimmune hepatitis, primary biliary cirrhosis, hemochromatosis and Wilson disease.

In one embodiment, the subject is a cirrhotic patient. In one embodiment, the patient was previously diagnosed as cirrhotic by any method known in the art, including invasive (e.g. biopsy) or non-invasive (e.g. blood test or physical method) methods already disclosed in the art.

In one embodiment of the invention, the mathematical combination is a combination within a mathematical function selected from a binary logistic regression, a multiple linear regression or any multivariate analysis. One skilled in the art may found in the prior art all information related to the mathematical function.

In one embodiment, the mathematical function is a logistic regression. A logistic regression produces a formula in the form:

$$score = a_0 + a_1 x_1 + a_2 x_2 + \ldots$$

wherein the coefficients $a_i$ are constants and the variables $x_i$ are the variables (preferably independent variables).

Preferably, the mathematical function is a binary logistic regression where final score is $1/1-e^{score}$.

In one embodiment, the diagnostic method of the invention presents:

- a NPV (or sensitivity) of at least about 75%, preferably of at least about 80%, more preferably of at least about 85%, 86%, 87%, 88%, 89%, 90%, 91%, 92%, 93%, 94%, 95%, 96%, 97%, 98%, 99% or more, and/or
- a PPV (or specificity) of at least about 75%, preferably of at least about 80%, preferably of at least about 85%, 86%, 87%, 88%, 89%, 90%, 91%, 92%, 93%, 94%, 95%, 96%, 97%, 98%, 99% or more.

In one embodiment, the diagnostic method of the invention presents a NPV of at least 95% and/or a PPV of at least 90%.

In one embodiment, the diagnostic method of the invention presents a diagnostic performance (patients correctly classified or AUROC) for esophageal varices, preferably for large esophageal varices, of at least about 0.89, preferably of at least about 0.90, 0.91, 0.92, 0.93, 0.94, 0.95, 0.96, 0.97, 0.98, 0.99 or more.

In one embodiment, the percentage of correctly classified patients using the method of the invention is of at least about 90%, preferably of at least about 90.5, 91, 91.5, 92, 92.5, 93, 93.5, 94, 94.5, 95, 95.5, 96, 96.5, 97, 97.5, 98, 98.5, 99, 99.5 or more.

In one embodiment, the diagnostic method of the invention presents a specificity of at least 90%, preferably of at least 91, 92, 93, 94, 95, 96, 97, 98, 99% or more. In one embodiment, the diagnostic method of the invention presents a specificity of 100%.

In one embodiment, using the diagnostic method of the invention, an invasive test for determining the presence or absence of esophageal varices, such as, for example, endoscopy (UGEI) is required in at most about 50 %, preferably in at most about 45, 40, 35, 30, 25, 20, 15, 10% or less of the hepatic disease patients.

In one embodiment, using the diagnostic method of the invention, the rate of saved UGEI is of at least about 20%, preferably of at least about 30, 40, 50, 60, 70, 80, 90% or more

In one embodiment, using the diagnostic method of the invention, the rate of missed large esophageal varices is of at most about 20%, preferably of at most about 19, 18, 17, 16, 15, 14, 13, 12, 11, 10, 9, 8, 7, 6, 5, 4, 3, 2, 1% or less.

Another object of the invention is a non-invasive method for assessing the presence and/or severity of varices, selected from gastric and esophageal varices in a liver disease patient, preferably in a patient with chronic liver disease, wherein said method comprises:

i. measuring at least one of the following variables from the subject:

- biomarkers,
- clinical data,
- binary markers,
- physical data from medical imaging or clinical measurement

ii. obtaining imaging data on varices status, wherein said imaging data are obtained by a non-invasive imaging method,
iii. mathematically combining, preferably in a binary logistic regression,

- the variables obtained in step (i), or any mathematical combination thereof with
- the data obtained at step (ii),

wherein the mathematical combination results in a diagnostic score, and
iv. assessing the presence and/or severity of varices, selected from gastric and esophageal varices based on the diagnostic score obtained in step (iii).

In one embodiment, the biomarkers, clinical data, binary markers, physical data and imaging data on varices status are as defined hereinabove.

In one embodiment, the variables measured in step (i) are the variables of a CirrhoMeter (such as, for example, a CirrhoMeter$^{V2G}$ or a CirrhoMeter$^{V3G}$, preferably a CirrhoMeter$^{V2G}$).

In another embodiment, the variables measured in step (i) are the variables of a FibroMeter (such as, for example, a FibroMeter$^{V2G}$ or a FibroMeter$^{V3G}$).

In one embodiment, the imaging data are obtained in step (ii) by ECE.

In one embodiment, the variables obtained in step (i) are mathematically combined in a non-invasive diagnostic test, preferably in a score, prior to the mathematical combination with the data obtained at step (ii). In one embodiment, the variables obtained in step (i) are mathematically combined in a FibroMeter or in a CirrhoMeter.

In one embodiment, the step (iii) of the method of the invention comprises mathematically combining a CirrhoMeter (such as, for example, a CirrhoMeter$^{V2G}$ or a CirrhoMeter$^{V3G}$, preferably a CirrhoMeter$^{2G}$) or the variables of a CirrhoMeter (such as, for example, a CirrhoMeter$^{V2G}$ or a CirrhoMeter$^{V3G}$, preferably a CirrhoMeter$^{2G}$) with a data obtained by ECE.

In one embodiment, the step (iii) of the method of the invention comprises mathematically combining a FibroMeter (such as, for example, a FibroMeter$^{V2G}$ or a FibroMeter$^{V3G}$) or the variables of a FibroMeter (such as, for example, a FibroMeter$^{V2G}$ or a FibroMeter$^{V3G}$) with a data obtained by ECE.

In one embodiment, the patient was previously diagnosed with a cirrhosis.

In one embodiment, the patient was classified in the indeterminate zone according to the step (b) of the method as defined hereinabove.

In one embodiment of the invention, the method of the invention is computer implemented.

The present invention thus also relates to a microprocessor comprising a computer algorithm carrying out the prognostic method of the invention.

The skilled artisan would easily deduce that the method of the invention being indicative of the presence of varices, selected from gastric and esophageal varices, especially of large esophageal varices, it may be used by the physician willing to provide the best medical care to his/her patient. For example, a patient presenting varices, selected from gastric and esophageal varices will require treatment of said varices, while a patient without esophageal varices will be subjected to yearly surveillance of varices. On the other hand, a patient diagnosed in the indeterminate zone regarding the value of the diagnostic score of the invention may require an UGEI endoscopy in order to assess the presence or absence of varices.

Therefore, the present invention also relates to a method for adapting the treatment, the medical care or the follow-up of a patient, wherein said method comprises implementing the non-invasive method of the invention.

The present invention also relates to a method for monitoring the treatment of a patient, wherein said method comprises implementing the non-invasive method of the invention, thereby assessing the appearance of esophageal varices in a patient.

The present invention also relates to a method for treating a hepatic disease patient, wherein said method comprises (i) implementing the non-invasive method of the invention and (ii) treating the patient according to the value obtained by the patient.

**BRIEF DESCRIPTION OF THE DRAWINGS**

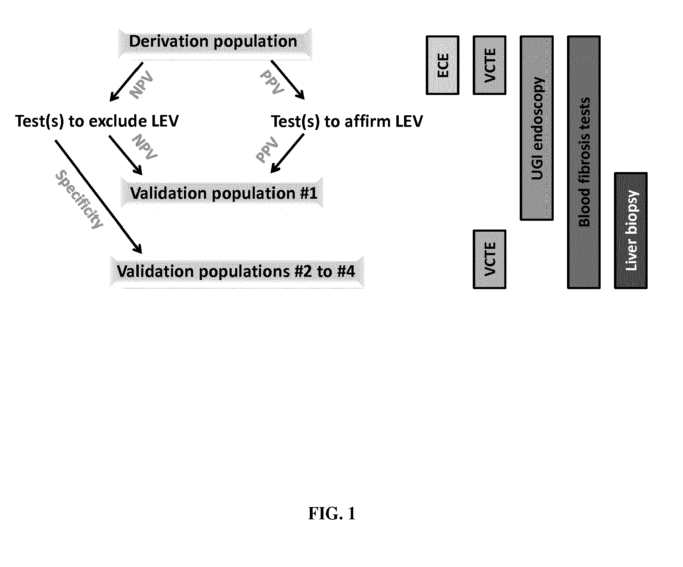

**Figure 1** is a graphic representation of the study design. Roles (oblique grey characters) of populations (horizontal bars), main investigations performed (vertical bars) and objectives (horizontal black characters). ECE: esophageal capsule endoscopy, LEV: large esophageal varices, NPV: negative predictive value, PPV: positive predictive value, UGI: upper gastro-intestinal, VCTE: vibration control transient elastography.

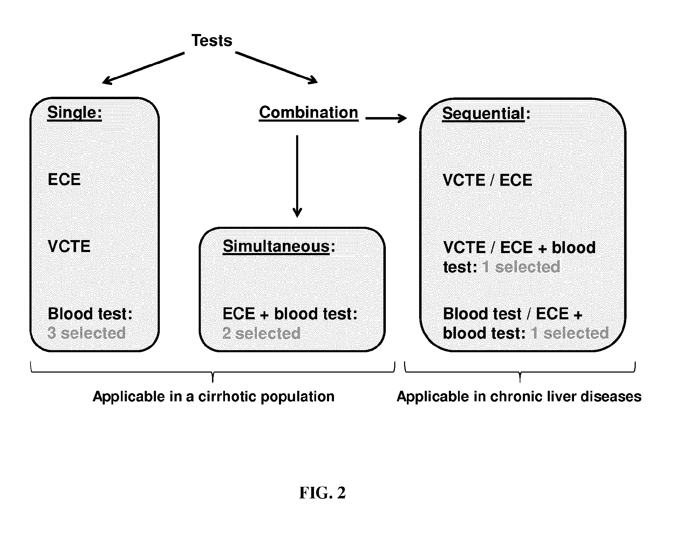

**Figure 2** is a graphic representation of the different strategies evaluated for LEV diagnosis. Among combinations, there were several possibilities but only the most clinically relevant were selected for evaluation (see table 6). ECE: esophageal capsule endoscopy, VCTE: vibration control transient elastography (Fibroscan).

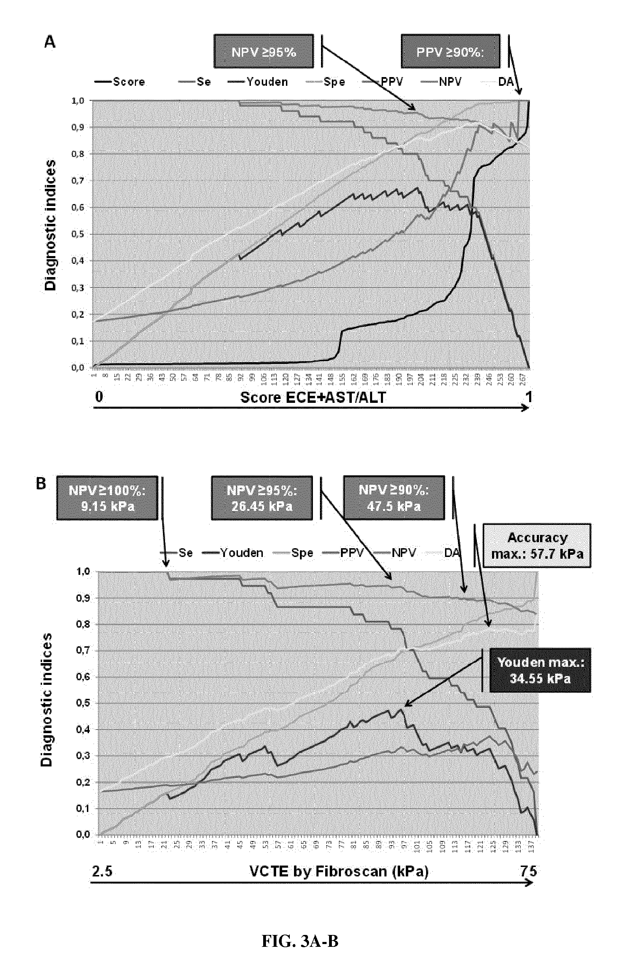

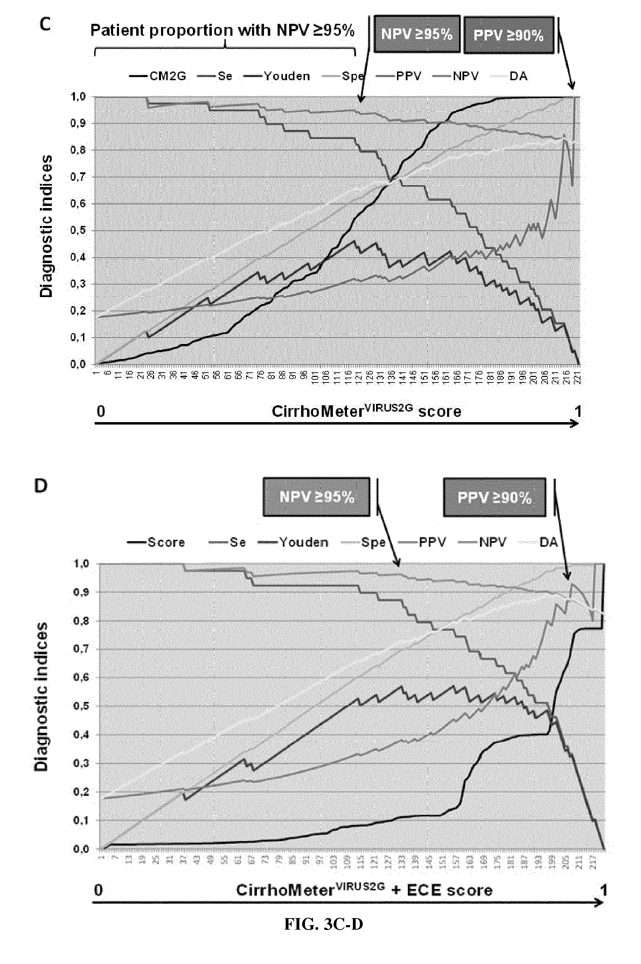

**Figure 3** is a combination of graphs showing diagnostic indices of non-invasive tests for large esophageal varices in derivation population. Two opposite examples: panel A shows the best score with large (in terms of patient proportion) zones of negative (NPV in light blue)) and positive predictive (PPV in dark green) values at 100%. Panel B shows VCTE (Fibroscan) with a low maximum PPV (<40%), i.e. no clinically interesting PPV zone. Panels C and D show the scores of the best clinically applicable strategy; note that the combination markedly improved the NPV$\geq$95% zone and the PPV$\geq$90% zone compared to CirrhoMeter$^{VIRUS2G}$ score. Se: sensitivity, Spe: specificity, DA: diagnostic accuracy, ECE: esophageal capsule endoscopy. Vertical figures on X axis indicate ranked patient values.

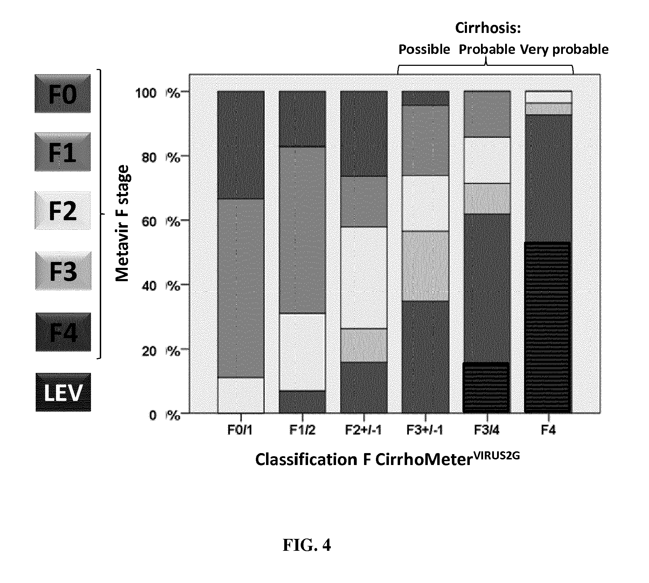

**Figure 4** is a histogram showing the relationship between CirrhoMeter$^{VIRUS2G}$ fibrosis classes (X axis), Metavir fibrosis (F) stages and large esophageal varices (Y axis) in validation population #1 with chronic liver disease. Note that LEVs were only present in Metavir F4 stage and that LEVs were more frequent in Metavir F4 classified as F4 than F3/4 by CirrhoMeter$^{VIRUS2G}$.

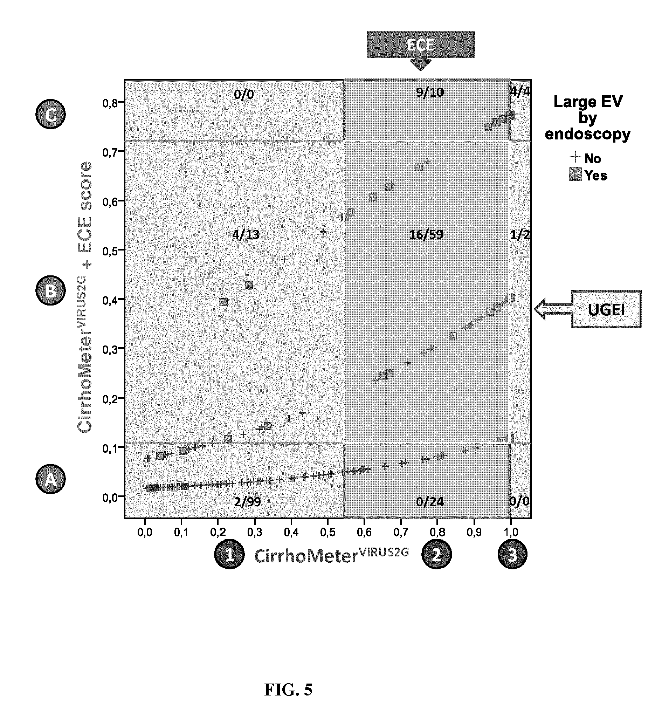

**Figure 5** is a scatter plot of CirrhoMeter$^{VIRUS2G}$ score (X axis) with CirrhoMeter$^{VIRUS2G}$ +ECE score (Y axis) as a function of LEV by endoscopy (UGEI) in the derivation population. The three curves are determined by ECE: no EV in bottom curve, small EV in intermediate curve and large EV in top curve. This figure clearly indicates that two patients with LEV without EV on ECE are rescued by the combination of CirrhoMeter$^{VIRUS2G}$ to ECE (lower right corner of zone 2B). Each score is divided into 3 zones according to high predictive value cut-offs. In VariScreen algorithm, CirrhoMeter$^{VIRUS2G}$ is performed first. ECE is then performed in indeterminate CirrhoMeter$^{VIRUS2G}$ zone 2. Thus, VariScreen zones are: LEV absence = zones 1 + 2A, LEV presence = zones 3 + 2C. Then, UGEI is performed in indeterminate VariScreen zone 2B. Figures x/y denote number of patients with LEV among all patients in each of the 9 zones determined by combination of the two tests.

**Figure 6** shows the VariScreen algorithm for large esophageal varices according to the present study. CirrhoMeter$^{VIRUS2G}$ is performed in all patients. Those below the CirrhoMeter$^{VIRUS2G}$ NPV cut-off for large EV have a 98-99% NPV for LEV. Those beyond the CirrhoMeter$^{VIRUS2G}$ PPV cut-off for large EV have a 83% PPV for LEV. Those patients between the two CirrhoMeter$^{VIRUS2G}$ cut-offs are offered ECE. Then, the ECE+CirrhoMeter$^{VIRUS2G}$ score is calculated in previous selected patients. Those below the NPV cut-off for LEV of ECE+CirrhoMeter$^{VIRUS2G}$ score have a 98-99% NPV for LEV. Those beyond the score PPV cut-off have a 90% PPV for LEV (detail not shown). Those patients between the two score cut-offs are offered endoscopy. ECE: esophageal capsule endoscopy, EV: esophageal varices.

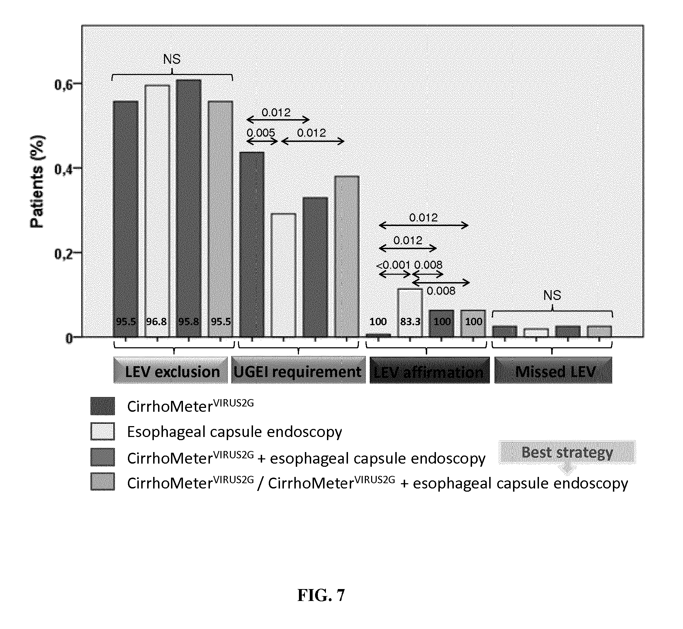

**Figure 7** is a histogram comparing all 4 strategies based on esophageal capsule endoscopy and/or CirrhoMeter$^{VIRUS2G}$ in derivation population. Figures inside bars indicate measured predictive values; figures above arrows indicate p value. Arrows indicate significant pairwise differences. Missed LEV are expressed here in proportion of all patients. LEV: large esophageal varices, UGIE: upper gastro-intestinal endoscopy, NS: not significant.

## EXAMPLES

The present invention is further illustrated by the following examples.

*PATIENTS AND METHODS*

**Patient populations**

Diagnostic strategy development needed a derivation population where all diagnostic tests were available, especially esophageal capsule endoscopy (ECE). For that purpose, we disposed of a population with cirrhotic patients. The derivation population was extracted from a prospective study comparing ECE and upper gastro-intestinal endoscopy (UGIE) in the esophageal varices (EV) diagnosis in patients with cirrhosis with various causes (Sacher-Huvelin S, et al, Endoscopy 2015:(in press: PMID: 25730284)). We included the 287 patients having both ECE and UGIE.
Validation populations were already published for the evaluation of non-invasive fibrosis tests (except population #4). The two main differences with derivation population were (i) the availability of liver biopsy in all patients and (ii) that all fibrosis stages were represented.

Validation of diagnostic strategy required a chronic liver disease (CLD) population with UGEI for reference and blood tests. Briefly, this validation population #1 was particular for several reasons. Patients with CLD attributed to virus or alcohol could have decompensated cirrhosis and had UGIE even in non-cirrhotic patients (Oberti F et al, Gastrointest Endosc 1998;48:148-157). Finally, we considered 3 additional large validation CLD populations #2 to #4 to mainly validate specificity robustness. Validation populations #2 (Pascal JP et al, N Engl J Med 1987;317:856-861) and #3 (Castera L et al, J Hepatol 2008;48:835-847) comprised patients with CLD due to chronic hepatitis C (CHC) without liver complication.

Population #4 comprised patients with CLD due to non-alcoholic fatty liver disease (NAFLD) without liver complication. Patients with biopsy-proven NAFLD were consecutively included in the study from January 2004 to June 2014 at Angers University Hospital and from October 2003 to April 2014 at Bordeaux University Hospital. NAFLD was defined as liver steatosis on liver biopsy after exclusion of concomitant steatosis-inducing drugs, excessive alcohol consumption (>210 g/week in men or >140 g/week in women), chronic hepatitis B or C infection, and histological evidence of other concomitant chronic liver disease. Patients were excluded if they had cirrhosis complications (ascites, variceal bleeding, systemic infection, or hepatocellular carcinoma). The study protocol conformed to the ethical guidelines of the current Declaration of Helsinki and all patients gave informed written consent.

**Study design**

Study design is summarized in **table 1** and **figure 1.**

Table 1. Main characteristics of populations.

|  | Derivation | Validation | | | |
|---|---|---|---|---|---|
|  |  | #1 | #2 | #3 | #4 |
| Cause | Miscellaneous | Alcohol, virus | Virus C | Virus C | NAFLD |
| Fibrosis spectrum | Cirrhosis | All stages | All stages | All stages | All stages |
| Endoscopy | **Yes** | **Yes** | No | No | No |
| ECE | **Yes** | No | No | No | No |
| Blood tests | **Yes** | **Yes** | **Yes** | **Yes** | **Yes** |
| VCTE | **Yes** | No | No | **Yes** | **Yes** |
| Liver biopsy | No | **Yes** | **Yes** | **Yes** | **Yes** |

*Diagnostic algorithms*

The diagnostic algorithms included different strategies (**figure 2**).

The first ones comprised a single diagnostic test. The second ones combined several tests. These combinations were either symmetric, i.e. the same test combination for both predictive values (PV), or asymmetric to reach higher PV, i.e. with different tests for negative predictive value (NPV) and positive predictive value (PPV). We defined a clinically applicable strategy as including necessarily a low constraint test (e.g. blood test) to exclude LEV (usually in asymptomatic patient) and possibly a high constraint test (e.g. UGIE) to affirm LEV (in the most severe patients).

*Strategy selection*

*First step* - We evaluated available strategies combining one or several tests to determine the most accurate strategy in the derivation population irrespective of clinical applicability in order to determine the most accurate strategy as paradigm.

*Second step* - We concentrated on the sole clinically applicable asymmetric strategies. When we compared strategies, we had no single comparator and a choice had to be based on a balance between the best three indicators (patient proportion with PV, saved UGEI and missed LEV, see below) according to statistical comparisons.

**Diagnostic tools**

*Eudoscopic procedures*

Endoscopic procedures of derivation population are detailed in our previous publication (Sacher-Huvelin S et al, Endoscopy 2015). In validation population #1, UGIE was performed by two senior endoscopists experienced in studies on PHT (Oberti F et al, Gastrointest Endosc 1998;48:148-157). In this study, EV size was also classified qualitatively into 3 grades: 1: small, 2: medium or 3: large. Stages 2 and 3 were grouped in LEV (Pascal JP et al, N Engl J Med 1987;317:856-861).

***Blood tests and elastometry***

*Blood tests* - The following blood tests were calculated according to published or patented formulas. Hepascore (Adams LA et al, Clin Chem 2005;51:1867-1873), Fib-4 (Sterling RK et al, Hepatology 2006;43:1317-1325), APRI (Wai CT et al, Hepatology 2003;38:518-526). FibroMeter[VIRUS2G] (Leroy V et al, Clin Biochem 2008;41:1368-1376), CirrhoMeter[VIRUS2G] (Boursier J et al, Eur J Gastroenterol Hepatol 2009;21:28-38), FibroMeter[VIRUS3G] (Calès P et al, J Hepatol 2010;52:S406) and CirrhoMeter[VIRUS3G] (Calès P et al, J Hepatol 2010;52:S406) were constructed for Metavir fibrosis staging in CHC. In FibroMeter /CirrhoMeter[VIRUS3G] GGT replaces hyaluronate included in FibroMeter/CirrhoMeter[VIRUS2G]. CirrhoMeter tests were constructed for cirrhosis diagnosis and included all FibroMeter markers ((Boursier J et al, Eur J Gastroenterol Hepatol 2009;21:28-38). FibroMeter[ALD] (Cales P et al, Gastroenterol Clin Biol 2008;32:40-51) and FibroMeter[NAFLD] (Cales P et al, J Hepatol 2009;50:165-173) were constructed for Metavir fibrosis staging, respectively in alcoholic liver disease (ALD) and NAFLD. QuantiMeter[NAFLD] was constructed to evaluate the area of whole fibrosis in NAFLD (Cales P et al, Liver Int 2010;30:1346-1354). QuantiMeter[VIRUS] and QuantiMeter[ALD] were constructed to evaluate the area of whole fibrosis in CHC and ALD, respectively (Cales P, et al, Hepatology 2005;42:1373-1381). All blood assays were performed in the same laboratories of each center, or partially centralized in population #3. Tests were used as raw data without correction rules like expert system.

*Elastometry* - Vibration control transient elastography (VCTE) (Fibroscan™, Echosens, Paris, France) examination was performed by an experienced observer (>50 examinations before the study), blinded for patient data. Examination conditions were those recommended by the manufacturer (Castera L et al, J Hepatol 2008;48:835-847). VCTE examination was stopped when 10 valid measurements were recorded. Results (kilopascals) were expressed as the median and the interquartile range of all valid measurements.

*Combined test* - One test combined markers of blood test and VCTE: Elasto-FibroMeter$^{2G}$ (E-FibroMeter$^{2G}$) (Cales P et al, Liver international: official journal of the International Association for the Study of the Liver 2014;34:907-917).

**Statistics**

***Diagnostic test segmentation***

For LEV diagnosis, we calculated diagnostic indices as a function of test values (**figure 3**).

We determined the cut-off of test value to reach NPV≥95% and a PPV≥90% in the largest subpopulation when possible. PPV and NPV were reported through two statistical descriptors. First, the patient proportion (% out of the whole population) being included between the first test value reaching the expected predefined cut-off (95 or 90%) and the extreme test value, called *PV patient proportion* thereafter (see **figure 3C**). Second, the *measured PV* (%) was determined in this patient group. All test cut-offs for LEV were derived from derivation population and test accuracy was validated in validation populations by using the same cut-offs. Finally each test included three zones from the lowest to highest values: LEV exclusion, indeterminate, LEV affirmation.

***Clinical descriptors***

*UGIE requirement* - This is the patient proportion in the indeterminate zone between NPV and PPV cut-offs for LEV.

*Missed LEV* - This is the proportion of LEV in the NPV zone for LEV.

*Saved UGIE* - The reference patient group to calculate saved UGEI is the cirrhosis group where UGEI is classically performed: the whole population in derivation population and patients with Metavir F4 stage in validation population #1. The saved UGEI rate is the patient proportion provided by the difference between the reference group and the target group where UGEI is indicated by non-invasive tests. The target group can be determined by cut-offs of fibrosis staging or LEV diagnosis.

***Statistical descriptors and tests***

Quantitative variables were expressed as mean $\pm$ standard deviation. 95% confidence intervals (CI) were calculated by bootstrapping on 1000 samples. The discriminative ability of each test was expressed as the area under the receiver operating characteristic (AUROC) curve and compared by the Delong test. Data were reported according to STARD (Bossuyt PM et al, Clin Chem 2003;49:7-189) and Liver FibroSTARD (Boursier J et al, J Hepatol 2015) statements, and analyzed on an intention to diagnose basis. Scores including independent predictors of LEV were determined by binary logistic regression. In the population where test is constructed, its accuracy is maximized and thus includes an optimism bias. Therefore, this bias was noticed when present. The main statistical analyses were performed under the control of professional statisticians using SPSS version 18.0 (IBM, Armonk, NY, USA) and SAS 9.2 (SAS Institute Inc., Cary, NC, USA).

*RESULTS*

**Population characteristics**

Characteristics of main populations are described in table 2 and those of ancillary validation populations in table 3.

**Table 2.** Patient characteristics in the two main populations (with UGEI).

| Characteristic | Population | |
| --- | --- | --- |
| | Derivation | Validation #1 |
| n patients | 287 | 165 |
| Sex (M %) | 72.1 | 64.2 |
| Age (yr) | 55.4±10.7 | 50.1±12.0 |
| BMI (kg/m$^2$) | 27.2± 5.6 | 24.0±4.2 |
| Cause (%): | | |
|   Alcohol | 64.5 | 72.7 |
|   Virus | 25.8 | 26.7 |
|   NAFLD | 5.6 | - |
|   Others | 4.2 | 0.6 |
| Metavir F: | | |
|   0 | 0 | 8.5 |
|   1 | 0 | 19.4 |
|   2 | 0 | 14.5 |
|   3 | 0 | 6.7 |
|   4 | 100[a] | 50.9 |
|   Score | 4 | 2.7±1.5 |
| Child-Pugh class: | | |
|   A | 60.3 | 72.6 (54.8)[b] |
|   B | 20.6 | 14.4 (23.8) |
|   C | 19.1 | 13.0(21.4) |
| Child-Pugh score: | 6.7±2.5 | 6.3±2.2 (7.2±2.5) |
| FibroMeter$^{VIRUS2G}$ | 0.82±0.21 | 0.74±0.28 (0.94±0.10) |
| Liver stiffness (kPa) | 33.4 ± 23.6 | - |
| Esophageal varices by endoscopy / ECE (%): | | |
|   No | 55.7 / 58.9 | 59.4 (29.8) / - |
|   Small | 26.8 / 28.9 | 18.8 (27.4) / - |
|   Large | 17.4 / 12.2 | 21.8 (42.9) / - |

BMI: body mass index, ECE: esophageal capsule endoscopy, NA: not available

[a] Estimation

[b] In cirrhosis in brackets

Table 3. Patient characteristics in the 3 ancillary validation populations.

| | Population | | |
| Characteristic | #2 | #3 | #4 |
|---|---|---|---|
| n patients | 1013 | 712 | 520 |
| Sex (M %) | 59.6 | 61.1 | 63.3 |
| Age (yr) | 45.4±12.5 | 51.7±11.2 | 54.5±13.0 |
| Body mass index (kg/m$^2$) | NA | 25.2±4.6 | 29.6±6.0 |
| Cause (%): | Virus | Virus | NAFLD |
| Metavir F stage: | | | |
| 0 | 4.3 | 3.8 | 23.3 |
| 1 | 43.3 | 37.8 | 31.5 |
| 2 | 27.0 | 25.7 | 19.2 |
| 3 | 13.9 | 17.8 | 16.3 |
| 4 | 11.4 | 14.9 | 9.6 |
| Score | 1.8±1.1 | 2.0±1.1 | 1.6±1.3 |
| Child-Pugh class in F4: | A | A | A |
| FibroMeter[VIRUS2G] | 0.50±0.31 | 0.60±0.28 | 0.48±0.28 |
| Liver stiffness (kPa) | - | 10.0±7.9 | 12.6±11.3 |
| NA: not available | | | |

Differences between populations were observed with respect to etiology and severity of liver disease and also concerning the investigations performed (**table 1**). In validation CLD population #1, LEV were only observed in patients with confirmed cirrhosis according to liver biopsy and in those with probable cirrhosis according to CirrhoMeter[VIRUS2G] **(figure 4).**

**<u>Overall accuracy of tests</u>**

Accuracies by AUROC of predictors for LEV are detailed in **table 4.**

**Table 4**. AUROC for large esophageal varices. Markers are ranked by increasing order in the derivation population with common size. Colors distinguish 0.1 intervals in AUROC.

| Marker | Population | | | |
| --- | --- | --- | --- | --- |
| | Derivation | | | Validation #1 [b] |
| | Maximum size | | Common size | |
| | N patients | AUROC (95% CI) | AUROC [a] | |
| 1. Age | 287 | 0.501 (0.414-0.589) | 0.443 | 0.707 |
| 2. Spleen diameter | 119 | 0.518 (0.400-0.637) | 0.508 | 0.697 |
| 3. ALT | 287 | 0.532 (0.445-0.619) | 0.516 | 0.705 |
| 4. Leucocytes | 283 | 0.527 (0.436-0.617) | 0.522 | - |
| 5. Body mass index | 270 | 0.479 (0.396-0.561) | 0.526 | 0.630 |
| 6. GGT | 287 | 0.582 (0.500-0.664) | 0.526 | 0.562 |
| 7. Alpha2-macroglobulin | 248 | 0.524 (0.436-0.612) | 0.529 | 0.656 |
| 8. Weight | 278 | 0.491 (0.414-0.567) | 0.531 | 0.592 |
| 9. Segmented leucocytes | 216 | 0.578 (0.476-0.679) | 0.534 | - |
| 10. Height | 273 | 0.568 (0.484-0.652) | 0.563 | 0.403 |
| 11. Monocytes | 216 | 0.565 (0.459-0.671) | 0.571 | - |
| 12. Hemoglobin | 284 | 0.661 (0.578-0.744) | 0.629 | 0.654 |
| 13. P2/MS | 216 | 0.619 (0.515-0.722) | 0.632 | - |
| 14. Alphafoeto protein | 261 | 0.595 (0.510-0.680) | 0.646 | - |
| 15. Alkaline phosphatases | 282 | 0.652 (0.578-0.726) | 0.647 | - |
| 16. Sodium | 263 | 0.639 (0.557-0.721) | 0.674 | 0.521 |
| 17. Platelets | 284 | 0.630 (0.536-0.725) | 0.675 | 0.769 |
| 18. AST | 287 | 0.646 (0.570-0.721) | 0.681 | 0.494 |
| 19. InflaMeter | 246 | 0.642 (0.556-0.727) | 0.684 | - |
| 20. Creatinine | 283 | 0.610 (0.525-0.694) | 0.686 | 0.523 |
| 21. Urea | 279 | 0.681 (0.594-0.767) | 0.698 | 0.536 |
| 22. APRI | 284 | 0.655 (0.576-0.733) | 0.704 | 0.682 |
| 23. Child-Pugh score | 287 | 0.718 (0.640-0.796) | 0.718 | 0.782 |
| 24. Fib-4 | 284 | 0.702 (0.625-0778) | 0.725 | 0.790 |
| 25. VCTE | 211 | 0.738 (0.662-0.815) | 0.730 | - |

| 26. Albumin | 275 | 0.727 (0.655-0.799) | 0.734 | 0.743 |
| 27. FibroMeter for cause | 251 | 0.736 (0.667-0.805) | 0.736 | - |
| 28. AST/ALT | 287 | 0.737 (0.667-0.807) | 0.747 | 0.678 |
| 29. Prothrombin index | 284 | 0.733 (0.660-0.807) | 0.752 | **0.871** |
| 30. FibroMeter[VIRUS3G] | 243 | 0.755 (0.680-0.829) | 0.761 | **0.857** |
| 31. CirrhoMeter[VIRUS3G] | 243 | 0.752 (0.678-0.827) | 0.763 | **0.911** |
| 32. Bilirubin | 284 | 0.738 (0.670-0.806) | 0.771 | **0.817** |
| 33. Elasto-FibroMeter[VIRUS2G] | 160 | 0.775 (0.694-0.857) | 0.773 | - |
| 34. AST/ALT + prothrombin | 284 | 0.763 (0.693-0.834) | 0.778 | **0.836** |
| 35. Hyaluronate | 225 | 0.772 (0.703-0.842) | 0.794 | **0.852** |
| 36. AST/ALT + hyaluronate | 225 | 0.777 (0.702-0.853) | 0.794 | **0.810** |
| 37. QuantiMeter[VIRUS] | 210 | 0.707 (0.618-0.795) | 0.799 | **0.870** |
| 38. QuantiMeter for cause | 249 | 0.770 (0.701-0.839) | 0.799 | - |
| 39. CirrhoMeter[VIRUS2G] | 211 | 0.765 (0.683-0.847) | **0.800** | **0.911** |
| 40. Hepascore | 213 | 0.768 (0.693-0.842) | **0.801** | **0.863** |
| 41. FibroMeter[VIRUS2G] | 211 | 0.768 (0.686-0.850) | **0.810** | **0.884** |
| 42. EV stage by ECE (15 mm) | 287 | **0.874 (0.819-0.929)** | 0.845 | - |
| 43. EV stage by ECE (25 mm) | 287 | **0.885 (0.829-0.840)** | 0.867 | - |
| 44. ECE + CirrhoMeter[VIRUS2G] | 211 | **0.907 (0.861-0.952)** | 0.906 | - |
| 45. ECE + AST/ALT | 287 | **0.916 (0.876-0.957)** | 0.911 | - |

ECE: esophageal capsule endoscopy, EV: esophageal varices, VCTE: vibration control transient elastography. AUROCs >0.8 are shown in bold.

[a] 158 patients. [b] Validation population #1 including 165 patients

Briefly, in derivation population, the highest AUROC at 0.92 was obtained with ECE+(AST/ALT) score. This score and ECE+CirrhoMeter[VIRUS2G] score had significantly higher AUROC than fibrosis tests (p<0.02) whereas AUROCs between other fibrosis tests were not significantly different (data not shown). In validation population, AUROC of Metavir F stage was significantly inferior to that of the most accurate tests (CirrhoMeters and FibroMeter[VIRUS2G]). This suggests that non-invasive testing can be more effective for LEV diagnosis than a histological diagnosis of cirrhosis.

## Diagnostic strategies

**Strategy selection according to accuracy**

***LEV absence***

*Derivation population* - CirrhoMeter[VIRUS2G] was the most accurate low constraint test resulting in the highest NPV patient proportion and the highest measured NPV for LEV among all strategies (**table 5**).

**Table 5.** Different diagnostic strategies for LEV developed in derivation population (with common size: n= 158) according to high negative (absence) and positive (presence) predictive value zones for LEV. The indeterminate zone lies between the two previous zones and corresponds to endoscopy requirement. These figures are proportions among all patients. The rate of missed LEV is the proportion of patients with LEV in the absence zone among patients with LEV. Figures in brackets are 95% CI.

| Strategy | Large EV | | | |
|---|---|---|---|---|
| | Absence | Indeterminate | Presence | Missed |
| **Single test:** | | | | |
| ECE | | | | |
| Patients (%) | 59.5 (51.6-67/5) | 29.1[a] (21.5-36.1) | **11.4** (6.9-16.8) | **10.0** (0-22.2) |
| Predictive value (%) | **96.8 (92.9-100)** | - | *83.3*[b] (62.5-100) | - |
| VCTE | | | | |
| Patients (%) | 47.5 (40.0-55.7) | 52.5 (44.4-60.1) | *0*[c] (0-0) | 13.3 (2.7-26.1) |

(continued)

| Strategy | Large EV | | | |
|---|---|---|---|---|
| | **Absence** | **Indeterminate** | **Presence** | **Missed** |
| **Single test:** | | | | |
| Predictive value (%) (AST/ALT)+PI score | 94.7 (88.6-98.7) | - | - | |
| Patients (%) | 55.1 (46.7-62.3) | 44.3 (36.7-51.9) | 0.6(0-2.0) | 16.7 (3.7-30.8) |
| Predictive value (%) (AST/ALT)+hyaluronate score | 94.3 (89.3-98.8) | - | 100 (100-100) | - |
| Patients (%) | 54.4 (46.5-62.0) | 44.3 (35.5-51.9) | 1.3 (0-3.2) | *20.0 (6.1-35.1)* |
| Predictive value (%) CirrhoMeter^VIRUS2G | *93.0 (87.5-97.9)* | - | 100 (100-100) | - |
| Patients (%) | 55.7 (47.7-63.1) | 40.5 (33.1-48.1) | 3.8 (1.2-7.2) | 13.3 (3.0-27.3) |
| Predictive value (%) | 95.5 (90.4-99.0) | | 83.3 | (NA) |
| **Simultaneous combination:** | | | | |
| ECE+(AST/ALT) score | | | | |
| Patients (%) | **78.5** (72.1-84.8) | **11.4** (6.3-16.5) | 10.1 (5.7-15.1) | *26.7 (11.5-44.1)* |
| Predictive value (%) | 93.5 (88.9-97.6) | - | 93.8 (76.9-100) | - |
| ECE+CirrhoMeter^VIRUS2G score | | | | |
| Patients (%) | 58.9 (50.3-66.2) | 34.8 (27.6-42.6) | 6.3 (3.0-11.0) | 6.7 (0.0-16.7) |
| Predictive value (%) | 97.8 (94.7-100) | - | **100 (100-100)** | - |
| **Sequential combination:** | | | | |
| VCTE/ECE | | | | |
| Patients (%) | 47.5 (40.0-55.7) | 43.0 (35.4-51.3) | 9.5[d] (5.1-14.2) | 13.3 (2.7-26.1) |
| Predictive value (%) | 94.7 (88.7-98.7) | - | 93.3 (76.9-100) | - |
| VCTE/ ECE+(AST/ALT) score | | | | |
| Patients (%) | 47.5 (40.0-55.7) | 43.7 (36.1-51.9) | 8.9[e] (4.7-13.5) | 13.3 (2.7-26.1) |
| Predictive value (%) | 94.7 (89.0-98.8) | - | 92.9 (76.9-100) | - |
| CirrhoMeter^VIRUS2G / ECE+ CirrhoMeter^VIRUS2G score | | | | |
| Patients (%) | 65.8 (57.6-73.1) | 26.6 (19.7-33.5) | 7.6 (3.7-12.4) | 13.3 (3.0-27.3) |
| Predictive value (%) | 96.2 (92.0-99.1) | - | **91.7 (71.4-100)** | - |
| p[f] | <0.001 | <0.001 | <0.001 | 0.648 |

LEV: large esophageal varices, ECE: esophageal capsule endoscopy, PI: prothrombin index, VCTE: vibration control transient elastography. Best results are shown in bold and worst in italics per zone.

[a] Patients diagnosed with small EV by ECE

[b] It was not possible to reach the objective (≥90%) since this is semi-quantitative variable.

[c] Maximum PPV was < 40%.

[d] This figure is different from ECE alone since 3 patients with high LEV PPV with ECE had high LEV NPV with VCTE.

[e] This figure is different from ECE + AST/ALT score alone (simultaneous combination) since 2 patients with high LEV PPV with ECE + AST/ALT score had high LEV NPV with VCTE.

[f] By paired Cochran test for patient proportions. Useful pairwise comparisons are provided in the text.

In the largest sample size tested for CirrhoMeter^VIRUS2G (**table 6**) the measured PPV was 98% (95%CI: 95-100) in a patient proportion of 59% (53-66).

**Table 6.** Comparison of LEV prediction between all 4 strategies based on esophageal capsule endoscopy (ECE) and/or CirrhoMeter[VIRUS2G] (CM). Figures in brackets are 95% CI. Derivation population (211 patients).

| Strategy | Large EV | | |
|---|---|---|---|
| | **Absence** | **Indeterminate** | **Presence** |
| **1. ECE** | | | |
| Patients (%) | 58.8 (52.6-65.7) | 29.4 (23.3-35.5) | 11.8 (7.6-16.5) |
| Predictive value (%) | 97.6 (94.9-100) | - | 80.0 (61.9-95.0 |
| 2. CirrhoMeter[VIRUS2G] | | | |
| Patients (%) | 53.1 (46.7-60.1) | 44.1 (37.1-50.7) | 2.8 (0.9-5.2) |
| Predictive value (%) | 94.6 (89.9-98.3) | - | 83.3 (NA) |
| 3. ECE+CirrhoMeter[VIRUS2G] score | | | |
| Patients (%) | 58.3 (52.2-64.9) | 35.1 (28.5-40.9) | 6.6 (3.3-10.3) |
| Predictive value (%) | 98.4 (95.6-100) | - | 92.9 (75.0-100) |
| 4. CirrhoMeter[VIRUS2G] / ECE+ CirrhoMeter[VIRUS2G] score | | | |
| Patients (%) | 64.5 (57.9-71.1) | 28.0 (22.1-34.2) | 7.6 (4.1-11.5) |
| Predictive value (%) | 95.6 (92.1-98.5) | - | 87.5 (68.8-100) |
| Comparison[a] | | | |
| All 4 strategies | 0.001 | <0.001 | <0.001 |
| ECE vs CM | 0.188 | 0.001 | <0.001 |
| ECE vs ECE+CM | 1 | 0.096 | 0.001 |
| ECE vs CM/ECE+CM | 0.104 | 0.775 | 0.022 |
| CM vs ECE+CM | 0.099 | 0.009 | 0.039 |
| CM vs CM/ECE+CM | <0.001 | <0.001 | 0.002 |
| ECE+CM vs CM/ECE+CM | <0.001 | <0.001 | 0.500 |

NA: not available

[a] Between patients proportions by paired Cochran test between the 4 strategies or paired McNemar test for pairwise comparison

*Validation populations* - With regard to the low constraint tests in validation population #1 (**table 7**), the most performant was again CirrhoMeter[VIRUS2G] since providing the highest measured NPV for LEV - 99% (97-100) - in the highest NPV patient proportion - 60% (53-68) - (**table 8**).

**Table 7.** Rates (%) of LEV prediction by LEV cut-offs of blood tests -from derivation population applied to validation population #1- according to LEV or cirrhosis presence. Figures in brackets are 95% CI.

| Blood test | Large EV | | |
|---|---|---|---|
| | **Absence** | **Indeterminate (↘)** | **Presence** |
| **(AST/ALT)+PI score:** | | | |
| Predictive value[a] | 92.8 | - | **100** |
| Patient proportion: | | | |
| No LEV | 69.8 (↗) | 30.2 | **0 (↘)** |
| LEV | *19.4* (↘) | 77.8 | 2.8 (↗) |
| F0-3 | 91.4 (↗) | 8.6 | **0 (↘)** |
| F4 | 27.4 | 71.4 | 1.2 (↗) |
| All patients | 58.8 | 40.6 | 0.6 |
| | (51.3-66.3) | (33.0-48.2) | (0-1.9) |
| **(AST/ALT)+hyaluronate score:** | | | |
| Predictive value[a] | 97.6 | - | 66.7 |
| Patient proportion: | | | |

(continued)

| (AST/ALT)+hyaluronate score: | | | |
|---|---|---|---|
| No LEV | 64.3 (↗) | 34.9 | *0.8* (↘) |
| LEV | 5.6[b] (↘) | 88.9 | **5.6** (↗) |
| F0-3 | 85.2 (↗) | 14.8 | 0 (↘) |
| F4 | 19.0 | 77.4 | **3.6** (↗) |
| All patients | 51.5 | 46.7 | 1.8 |
| | (43.9-59.0) | (39.3-54.4) | (0-4.2) |
| **CirrhoMeter[VIRUS2G]:** | | | |
| Predictive value[a] | **98.9** | - | - |
| Patient proportion: | | | |
| No LEV | **74.2** (↗) | **25.8** | **0** (↘) |
| LEV | **3.2** (↘) | 96.8 | **0** (↗) |
| F0-3 | **92.4** (↗) | **7.6** | **0** (↘) |
| F4 | 26.3 | 73.7 | *0* (↗) |
| All patients | 60.0 | 40.0 | 0 |
| | (52.6-67.7) | (32.3-47.4) | (0-0) |
| **Comparison [c]** | | | |
| Missed LEV | p=0.007 | - | - |
| UGEI requirement | - | p=0.030 | - |

LEV: large esophageal varices, PI: prothrombin index, F: Metavir fibrosis stage, UGIE: upper gastro-intestinal endoscopy. Best results are shown in bold and worst in italics per zone and patient category or predictive value. Arrows indicate the clinically suitable trends, e.g. LEV exclusion zone should be very low in no LEV or F0-3 patients and LEV affirmation zone high in LEV or F4 patients.
[a] Measured predictive value in all patients.
[b] Corresponds to missed LEV.
[c] By paired Cochran test between the 3 tests.

**Table 8.** Rates (%) of LEV prediction by CirrhoMeter[VIRUS2G] cut-offs for LEV -from derivation population- according to LEV or cirrhosis presence in CLD validation population #1. Figures in brackets are 95% CI. This table details some results of table 7.

| Blood test | Large EV | | |
|---|---|---|---|
| | **Absence** | **Indeterminate (↘)** | **Presence** |
| Predictive value : | | | |
| All patients | 98.9 (96.6-100) | - | - |
| No LEV | 100 (100-100) | - | - |
| LEV | 0 | - | - |
| F0-3 | 100 (100-100) | - | - |
| F4 | 59.2 (47.2-70.5) | - | - |
| Patient proportion: | | | |
| All patients | 60.0 (52.6-67.7) | 40.0 (32.3-47.4) | 0 |
| No LEV | 74.2 (67.4-81.5) (↗) | 25.8 (18.5-32.6) | 0 (↘) |
| LEV | 3.2 (0.0-10.3) (↘)[a] | 96.8 (89.7-100) | 0(71) |
| F0-3 | 92.4 (86.3-98.6) (↗) | 7.6 (1.4-13.8) | 0 (↘) |
| F4 | 26.3 (17.2-37.0) | 73.7 (63.0-82.8) | *0* (↗) |

LEV: large esophageal varices, PI: prothrombin index, F: Metavir fibrosis stage, UGIE: upper gastro-intestinal endoscopy. Arrows indicate the clinically suitable trends, e.g. LEV exclusion zone should be very low in no LEV or F0-3 patients and LEV affirmation zone high in LEV or F4 patients.
[a] Corresponds to missed LEV

In validation populations #2 to #4 (2245 CLD patients: **table 9**), CirrhoMeter[VIRUS2G] was the test with the highest NPV patient proportion (≥98%) in non-cirrhotic patients across the 3 populations.

**Table 9.** Robustness of cut-offs of blood tests for predictive values for LEV, as determined in the derivation population, in validation populations #2 to #4 (2245 CLD patients): patient proportion (%) as a function of cirrhosis (F4) presence. Results of validation population #1 are grouped in table 8.

| Fibrosis test | Validation population | | | | | | |
| --- | --- | --- | --- | --- | --- | --- | --- |
| | #2[a] | | #3[a] | | #4 | | |
| | NPV | Indet. | NPV | Indet. | NPV | Indet. | PPV |
| **AST/ALT+PI score:** | | | | | | | |
| F0-F3 | 98.9 | **1.1** | 98.5 | **1.5** | 96.6 | 3.4 | 0 |
| F4 | 77.4 | 22.6 | 91.5 | **8.5** | 74.0 | 26.0 | 0 |
| **AST/ALT+hyaluronate score**: | | | | | | | |
| F0-F3 | 97.3 | *2.7* | 95.2 | *4.8* | 90.9 | *9.1* | 0 |
| F4 | 55.7 | *44.3* | 68.9 | 31.1 | 56.0 | *42.0* | **2.0** |
| **CirrhoMeter[VIRUS2G]:** | | | | | | | |
| F0-F3 | 98.7 | **1.3** | 98.3 | **1.7** | 98.6 | **1.4** | 0 |
| F4 | 55.3 | *46.1* | 68.9 | 31.1 | 68.3 | 31.7 | 0 |
| **VCTE:** | | | | | | | |
| F0-F3 | - | - | 99.0 | **1.0** | 94.1 | 5.9 | 0 |
| F4 | - | - | 74.0 | *36.0* | 45.5 | *54.5* | 0 |

Indet.: indeterminate zone between NPV and PPV zones; NPV: negative predictive value zone, PPV: positive predictive value zone, PI: prothrombin index, VCTE: vibration control transient elastography. Clinically satisfactory results are shown in bold and those unsatisfactory are in italics.

[a] No PPV zone (0% patients)

*LEV presence*

*Derivation population* - Among the five single test strategies **(table 5),** ECE was the most accurate test due to a significantly lower indeterminate patient proportion (29%, p<0.001) and the highest PPV patient proportion (11%). However, the measured PPV for LEV in this subgroup did not reach the targeted value: 80% in the largest population **(table 6).** Among the five combination strategies, two strategies ranked first for the two PPV criteria. Thus, ECE+(AST/ALT) score had the highest PPV patient proportion (10%) but was hampered by a suboptimal measured PPV - 94% (77-100) - for LEV despite optimism bias. ECE+CirrhoMeter[VIRUS2G] score reached the highest measured PPV for LEV at the expense of a lower PPV patient proportion than in other combinations **(table 5).** Measured PPV was 93% (75-100) in a patient proportion of 7% (3-10) in the largest population **(table 6).**

*Validation populations* - In validation population #1 **(table 7),** the three available blood tests showed similar results to derivation population (i.e. a high PPV was only observed in ≤1% of patients despite a 22% LEV prevalence). Thus, blood tests alone are not sufficiently predictive of the presence of LEV.

*Most accurate strategy*

Among several clinically applicable strategies, the most accurate one seemed at this step to use first CirrhoMeter[VIRUS2G] mainly for LEV absence (i.e. the test with the highest NPV criteria), then to use the ECE+CirrhoMeter[VIRUS2G] score for LEV presence (i.e. a 93% PPV value in a substantial patient proportion). As there was a significant interaction (p<0.001) between CirrhoMeter[VIRUS2G] and ECE, we analyzed the plot of the two tests **(figure 5).** It clearly shows that UGEI has only to be required in the indeterminate zone common to the two tests which resulted in the proposed sequential diagnostic algorithm shown in **figure 6** and called VariScreen algorithm thereafter.

*Test robustness*

Robustness of test cut-off values for LEV prediction was evaluated in large unselected validation populations #2 to #4 (2245 CLD patients without decompensated cirrhosis) **(table 9).** Briefly, CirrhoMeter[VIRUS2G] robustness was validated, especially its estimated specificity was 100%, i.e. there was a priori no false positive LEV prediction in non-cirrhotic patients like in validation population #1.

**Strategy evaluation according to clinical aspects**

***LEV strategy comparison***

All strategies were compared within the derivation population **(table 5).** Among the 7 clinically applicable strategies, the most accurate were the 3 combined sequential strategies since the two clinical descriptors (UGEI requirement and missed LEV) were better or equal than in the 4 single test strategies. Among these 3 combined sequential strategies, that including CirrhoMeter[VIRUS2G] offered the advantage of a higher rate of saved ECE (i.e. NPV patient proportion) or UGEI than the two others (p<0.001) with similar missed EV rate. Finally, the two strategies combining CirrhoMeter[VIRUS2G] and ECE (simultaneous or sequential) were directly compared **(tables 6 and 10):** the simultaneous combination resulted in significant lower missed LEV rate. However, the sequential strategy significantly reduced UGEI requirement (-7%) while saving 65% ECE.

**Table 10.** Rates (%) of saved endoscopy and missed LEV by using two strategies based on CirrhoMeter[VIRUS2G] $\pm$ ECE. The first strategy (A) is the recent attitude of performing UGEI according to non-invasive fibrosis staging; the second strategy (B) is that developed in the present study based on non-invasive tests targeted for LEV. Figures in brackets are 95 % CI.

| Strategy | Saved endoscopy | Missed LEV |
|---|:---:|:---:|
| **Derivation population [a]:** | | |
| A. Cirrhosis staging by CM [b] followed by UGEI in: | | |
| Possible cirrhosis | 15.6 (10.7-20.5) | **2.8 (0.0-9.1)** |
| Probable cirrhosis | 36.5 (30.2-43.0) | 11.1 (2.3-21.4) |
| Very probable cirrhosis | **61.1 (54.5-67.4)** | *33.3 (18.2-50.0)* |
| p[c] | <0.001 | <0.001 |
| B. LEV prediction according to [d]: | | |
| CM | 55.9 (49.5-62.8) | 16.7 (4.6-29.4) |
| CM+ECE score | 64.9 (58.3-71.6) | **5.6 (0.0-14.3)** |
| CM / CM+ECE score | **72.0 (65.7-78.1)** | 16.7 (4.6-29.4) |
| p[e] | <0.001 | 0.018 |
| **Validation population #1:** | | |
| A. Cirrhosis staging by CM [b] followed by UGEI in: | | |
| Possible cirrhosis | *-28.9 (p<0.001) [f]* | 0 |
| Probable cirrhosis | 1.3 (p=1)[f] | **0** |
| Very probable cirrhosis | **28.9 (p<0.001)[f]** | 9.7 (0.0-21.9) |
| p[g] | <0.001 | 0.048 |
| B. LEV prediction according to [d] [h]: | | |
| CM | 18.4 (p=0.009)[f] | 3.2 (0.0-10.3) |
| CM / CM+ECE score | **48.2[i]** (p<0.001)[f] | **3.2 (0.0-10.3)** |

(continued)

| Strategy | Saved endoscopy | Missed LEV |
|---|---|---|
| p[k] | <0.001 | 1 |

ECE: esophageal capsule endoscopy, CM: CirrhoMeter[VIRUS2G], LEV: large esophageal varices, F: Metavir fibrosis stage. Satisfactory results are shown in bold and unsatisfactory in italics per population and strategy.

[a] The rates were calculated in the derivation population with maximum size (n=211)

[b] Cut-offs of CM classes are those defined a priori for fibrosis stages in previous publication (Cales P et al, Journal of clinical gastroenterology 2014): cirrhosis is defined as possible (classes F3±1, F3/4 and F4) or probable (classes F3/4 and F4) or very probable (classes F4). UGEI is performed only in the classes selected. The significance of gain could not be calculated since UGEI was performed in every patient.

[c] By paired Cochran test between the 3 proportions. Pairwise comparisons by paired McNemar test: saved UGEI: all three pairs: p<0.001; missed LEV: possible vs probable: p=0.250, possible vs very probable: p=0.001, probable vs very probable: p=0.008.

[d] Cut-offs of CirrhoMeter[VIRUS2G] classes and scores were defined a posteriori for LEV in the current derivation population. ECE is performed outside the PV zones of CM and UGEI is performed in the indeterminate zone.

[e] By paired Cochran test between the 3 proportions. Pairwise comparisons by McNemar test for saved UGEI: CM vs CM/CM+ECE: p=0.001, CM vs CM+ECE: p<0.001, CM/CM+ECE vs CM+ECE: p=0.038.

[f] Comparison vs UGEI in histological cirrhosis by paired McNemar test. 95%CI cannot be calculated since this figure is obtained by a proportion difference

[g] By paired Cochran test between the 3 proportions. Pairwise comparisons by McNemar test: saved UGEI: all three pairs: p<0.001; missed LEV: not calculable.

[h] The simultaneous strategy based on CirrhoMeter[VIRUS2G]+ECE score was not evaluated since clinically unsuitable in a CLD population

[i] Estimated calculation by applying the rate of saved UGEI by CM+ECE score in the indeterminate CM zone from derivation population (36.6%)

[k] By paired McNemar test

### *Comparison of direct LEV screening vs indirect fibrosis staging*

We compared the direct LEV screening developed in the present study vs an indirect screening based on cirrhosis diagnosis (reference for UGEI requirement in the present study) or non-invasive fibrosis staging. Thus, we compared the three strategies including CirrhoMeter[VIRUS2G] **(table 10).** Briefly, in the validation CLD population, a strategy of performing endoscopy by the possible cirrhosis class of CirrhoMeter[VIRUS2G] would induce a significant UGEI overuse of 29% compared to conventional histological diagnosis of cirrhosis. Finally, at similar missed LEV rate, the saved UGEI rate was much higher when CirrhoMeter[VIRUS2G] was targeted for LEV than for fibrosis, e.g. 18.4% (p=0.009) vs. 1.3% (p=1), respectively in derivation population.

### **Clinical improvement by sequential combination of CirrhoMeter[VIRUS2G] to ECE**

Direct comparison of CirrhoMeter[VIRUS2G], ECE and their combinations was performed in derivation population **(figure 7, table 6).**

CirrhoMeter[VIRUS2G] was as accurate as ECE to predict LEV absence. However, ECE was significantly more accurate than CirrhoMeter[VIRUS2G] to predict LEV presence. Sequential combination significantly decreased the patient proportion with LEV presence from 12 to 8% compared to simultaneous combination but this was counterbalanced by an increase in measured PPV from 83% to 88%. The UGEI requirement by this sequential combination was significantly reduced when compared to CirrhoMeter[VIRUS2G] but not significantly different compared to ECE. The missed EV rate was significantly decreased by simultaneous combination compared to other strategies only in the derivation population. Finally, the sequential combination spared 48 to 72% of UGEI, whether UGEI would have been performed in all patients with cirrhosis, and spared 65% of ECE, whether ECE would have been performed in all CLD. The rate of correctly classified patients for LEV was, CirrhoMeter: 96.7% ((94.3-99.0), ECE: 90.0% (86.1-93.9), (CirrhoMeter+ECE) score: 98.6% (96.7-100), VariScreen algorithm: 96.2% (93.1-98.6), p<0.001 by paired Friedman test (pairwise comparison: ECE significantly lower than other tests and other test not significantly different between each other).

### **Misclassified patients**

They were 21 patients (10.0%) misclassified for LEV by ECE; 5 were false positive LEV by ECE and 4 were rescued by VariScreen; there were 16 false negative LEV by ECE and 15 were rescued by VariScreen. Thus, 20 patients were rescued. However, there were 6 false negative LEV and 1 false positive LEV by VariScreen so that the net result was 20 - 7 = 13 corresponding to the 6.2% gain in accuracy by VariScreen compared to ECE. The 6 patients with missed LEV by VariScreen algorithm had blood markers significantly different (reflecting a better liver status) from other patients with LEV, e.g. serum albumin level (not included in CirrhoMeter): ruling out zone: $36.2\pm6.9$ g/l, indeterminate zone: $34.1\pm5.8$, ruling in zone: $25.9\pm5.1$, p<0.001 by ANOVA.

**DISCUSSION**

**Originalities**

The present study is the first one to compare ECE and fibrosis tests for the non-invasive diagnosis of LEV (Colli A et al, Cochrane Database Syst Rev 2014;10:CD008760). In addition, studies of non-invasive diagnosis of LEV were performed in patients with cirrhosis selected by other means than non-invasive fibrosis tests. This casts some uncertainty about the cut-off exportability of non-invasive test cut-offs for LEV diagnosis in populations where cirrhosis will be diagnosed by the same non-invasive tests (for fibrosis staging there) among CLD. Therefore, we also evaluated together these tests in a population of CLD including non-cirrhotic patients with available UGIE which is a unique population. The only diagnostic combination algorithm published for high-risk EV was a sequential algorithm based on liver stiffness and concordant blood test in a first step followed by spleen stiffness in the intermediate zone; but the accuracy was only around 77% (Stefanescu H et al, Liver Int 2014).

**Main results**

Among fibrosis tests, blood tests appeared more interesting than VCTE for LEV diagnosis. This is due not only to a low maximum PPV for LEV but also to a lesser NPV patient proportion with VCTE **(table 6).** VCTE has been shown to well diagnose PHT level (Bureau C et al, Aliment Pharmacol Ther 2008;27:1261-1268) but was limited and inferior to a single blood marker, like prothrombin index, for LEV diagnosis (Castera L et al, J Hepatol 2009;50:59-68).

Among single tests, ECE was the most accurate non-invasive diagnosis for LEV providing the lowest rates of endoscopy requirement and missed LEV **(table 5).** In clinical practice, we have to choose a sequential diagnostic strategy based on a low constraint test to exclude LEV (expectedly in non-cirrhotic CLD) and on the most accurate test to diagnose LEV (expectedly used in cirrhosis). The most accurate sequential strategy was the VariScreen algorithm **(figure 6)** both in validation and derivation populations with a rate of saved endoscopy of 48 to 72% and a rate of missed LEV of 3 to 17%, respectively. In practical terms, CirrhoMeter[VIRUS2G] is performed in all CLD patients. Patients with CirrhoMeter[VIRUS2G] below NPV LEV cut-off are followed-up with yearly testing. Those with CirrhoMeter[VIRUS2G] beyond PPV LEV cut-off are offered primary prophylaxis. Those between the two CirrhoMeter[VIRUS2G] LEV cut-offs are offered ECE. Then, the ECE+CirrhoMeter[VIRUS2G] score is calculated by computerization. Patients with a score below NPV LEV cut-off are followed-up with yearly CirrhoMeter[VIRUS2G] testing. Patients with a score beyond PPV LEV cut-off are offered primary prophylaxis, either pharmacological or endoscopic (which could be a preferable option to validate non-invasive diagnosis in rare cases without LEV on ECE). Patients between the two LEV cut-offs of ECE+CirrhoMeter[VIRUS2G] score are offered UGEI **(figure 6).**

Finally, this study confirms that the non-invasive cirrhosis diagnosis has the potential to induce a roughly 30% endoscopy overuse. But applying cut-off of single fibrosis test specific for LEV is able to save one out 5 endoscopies compared to conventional strategy with cirrhosis diagnosis determined by liver biopsy **(table 10).**

**Result comments**

The VariScreen Algorithm for LEV was not perfect with an indeterminate zone but it offered clinically relevant prediction with 88% PPV; moreover, the patients with false positive of VariScreen for LEV had small EV **(table 11).**

**Table 11.** Distribution of small EV as a function of VariScreen algorithm; patient number in derivation population (211 patients).

| UGEI | LEV | | |
|------|---------|---------------|----------|
| | **Absence** | **Indeterminate** | **Presence** |
| Esophageal varices: | | | |
| Absence | 98 | 17 | 0 |
| Small | 36 | 26 | 2 |
| Large | 6 | 16 | 14 |

In addition, 96-99% NPV and 100% specificity were obtained in substantial patient proportions. The two latter figures were obtained with CirrhoMeter[VIRUS2G] which is the only available test specifically designed for cirrhosis diagnosis. It includes, hyaluronate which was the most accurate blood marker for LEV in the present study and elsewhere, and platelets and prothrombin index that are known markers for LEV.

**Conclusion**

The non-invasive diagnosis of LEV exhaustively applied to CLD is superior to the conventional attitude based on liver biopsy or clinics followed by endoscopy in all patients with cirrhosis in terms of saved endoscopy. Likewise, the use of specific cut off for LEV of a blood test spared endoscopy compared to the strategy using cut-off of this blood test for cirrhosis followed by endoscopy. This sparing effect can be significantly improved by ECE. Therefore, in the era of non-invasive testing, tests should be primarily focused on cirrhosis complications screening, like LEV, rather on cirrhosis diagnosis itself.

**Claims**

1. A non-invasive method for assessing the presence and/or severity of varices, selected from gastric and esophageal varices in a liver disease patient, wherein said method comprises:

   (a) carrying out a non-invasive test for assessing the severity of a hepatic lesion or disorder, wherein said non-invasive test results in a value, and
   (b) comparing the value obtained at step (a) with cut-offs of said non-invasive test for assessing the presence and/or severity of varices, selected from gastric and esophageal varices.

2. The non-invasive method according to claim **1,** wherein the method is for assessing the presence of large esophageal varices.

3. The non-invasive method according to claim **1** or **2,** wherein said cut-offs are a NPV cut-off and a PPV cut-off, or a sensitivity cut-off and a specificity cut-off.

4. The non-invasive method according to claim **3,** wherein:

   - a value obtained in step (a) below the NPV cut-off or below the sensitivity cut-off is indicative of the absence of varices, selected from gastric and esophageal varices, preferably of large esophageal varices, in the patient, and
   - a value obtained in step (a) above the PPV cut-off or above the PPV cut-off is indicative of the presence of varices, selected from gastric and esophageal varices, preferably of large esophageal varices, in the patient.

5. The non-invasive method according to claim **3** or **4,** wherein, if the value obtained in step (a) is between the NPV cut-off and the PPV cut-off or between the sensitivity cut-off and the specificity cut-off, then the method further comprises the steps of:

(c) measuring at least one of the following variables from the subject:

    - biomarkers,
    - clinical data,
    - binary markers,
    - physical data from medical imaging or clinical measurement (d) obtaining imaging data on varices status, wherein said imaging data are obtained by a non-invasive imaging method,

(e) mathematically combining, preferably in a binary logistic regression,

    - the variables obtained in step (c), or any mathematical combination thereof with
    - the data obtained at step (d),

wherein the mathematical combination results in a diagnostic score, and
(f) assessing the presence and/or severity of varices, selected from gastric and esophageal varices, preferably of large esophageal varices, based on the diagnostic score obtained in step (e).

6. The non-invasive method according to claim **5,** wherein the imaging data on varices status are obtained by a non-invasive imaging method, preferably esophageal capsule endoscopy; or by a radiologic method, preferably a scanner.

7. The non-invasive method according to anyone of claims **1** to **6,** wherein the non-invasive test carried out in step (a) is a blood test, preferably selected from ELF, FibroSpect™, APRI, FIB-4, Hepascore, Fibrotest™, FibroMeter™, CirrhoMeter™, CombiMeter, Elasto-FibroMeter™, Elasto-Fibrotest, InflaMeter™; or a physical method, preferably selected from VCTE, ARFI, VTE, supersonic elastometry or MRI stiffness.

8. The non-invasive method according to claim **5,** wherein at step (c), the obtained variables are the variables of the non-invasive test carried out in step (a).

9. The non-invasive method according to anyone of claims **1** to **8,** wherein the non-invasive test carried out in step (a) is a CirrhoMeter.

10. The non-invasive method according to claims **5** to **9,** wherein the non-invasive test carried out in step (a) is a CirrhoMeter, and wherein the variables obtained at step (c) are the variables of a CirrhoMeter.

11. The non-invasive method according to anyone of claims **1** to **10,** wherein the patient is affected with a chronic hepatic disease, preferably selected from the group comprising chronic viral hepatitis C, chronic viral hepatitis B, chronic viral hepatitis D, chronic viral hepatitis E, non-alcoholic fatty liver disease (NAFLD), alcoholic chronic liver disease, autoimmune hepatitis, primary biliary cirrhosis, hemochromatosis and Wilson disease.

12. The non-invasive method according to anyone of claims **1** to **11,** wherein the patient is a cirrhotic patient.

13. A non-invasive method for assessing the presence and/or severity of varices, selected from gastric and esophageal varices, preferably of large esophageal varices, in a hepatic disease patient, wherein said method comprises:

i. measuring at least one of the following variables from the subject:

    - biomarkers,
    - clinical data,
    - binary markers,
    - physical data from medical imaging or clinical measurement,

ii. obtaining imaging data on varices status, wherein said imaging data are obtained by a non-invasive imaging method,
iii. mathematically combining, preferably in a binary logistic regression,

    - the variables obtained in step (i), or any mathematical combination thereof with

- the data obtained at step (ii),

wherein the mathematical combination results in a diagnostic score, and
iv. assessing the presence and/or severity of varices, selected from gastric and esophageal varices, preferably of large esophageal varices, based on the diagnostic score obtained in step (iii).

14. The non-invasive method according to claim **13,** wherein the patient was previously diagnosed as cirrhotic, or wherein the patient previously obtained a value between the NPV and the PPV cut-offs in a method according to claim **3.**

15. A microprocessor comprising a computer algorithm carrying out the method according to anyone of claims **1** to **14.**

FIG. 1

FIG. 2

**FIG. 3A-B**

FIG. 3C-D

FIG. 4

**FIG. 5**

**All patients with chronic liver disease**

↓

Blood testing by CirrhoMeter<sup>VIRUS2G</sup> score (0 to 1)

↓

Yearly surveillance
of test progression

Score ≤0.545
Score >0.9994
0.545< score ≤0.9994

No large EV

ECE

↓

Large EV

CirrhoMeter<sup>VIRUS2G</sup>+ECE score (0 to 1)

↓

Score ≤0.1117
Score >0.71
0.1117< score ≤0.71

↓

UGI endoscopy

**FIG. 6**

**FIG. 7**

Europäisches
Patentamt

European
Patent Office

Office européen
des brevets

# EUROPEAN SEARCH REPORT

Application Number

EP 15 16 2685

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2014/190170 A1 (UNIV CALIFORNIA [US]) 27 November 2014 (2014-11-27) * paragraph [0007] * ----- | 1-15 | INV. G01N33/68 G06F19/24 |
| X | ANTONIO COLECCHIA ET AL: "Measurement of Spleen Stiffness to Evaluate Portal Hypertension and the Presence of Esophageal Varices in Patients With HCV-Related Cirrhosis", GASTROENTEROLOGY, vol. 143, no. 3, 1 September 2012 (2012-09-01), pages 646-654, XP055194932, ISSN: 0016-5085, DOI: 10.1053/j.gastro.2012.05.035 * abstract * ----- | 1-15 | |
| X | DATABASE BIOSIS [Online] BIOSCIENCES INFORMATION SERVICE, PHILADELPHIA, PA, US; June 2001 (2001-06), COLLI AGOSTINO ET AL: "Renovascular impedance and esophageal varices in patients with Child-Pugh class A cirrhosis", XP055194869, Database accession no. PREV200100337764 * abstract * & RADIOLOGY, vol. 219, no. 3, June 2001 (2001-06), pages 712-715, ISSN: 0033-8419 ----- -/-- | 1-15 | TECHNICAL FIELDS SEARCHED (IPC) G01N G06F |

The present search report has been drawn up for all claims

| Place of search | Date of completion of the search | Examiner |
|---|---|---|
| Munich | 17 June 2015 | Hinchliffe, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 15 16 2685

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PILETTE C ET AL: "Non-invasive diagnosis of esophageal varices in chronic liver diseases", JOURNAL OF HEPATOLOGY, ELSEVIER, AMSTERDAM, NL, vol. 31, no. 5, 1 November 1999 (1999-11-01), pages 867-873, XP002373622, ISSN: 0168-8278, DOI: 10.1016/S0168-8278(99)80288-8 * see abstract, discussion and table 4 * | 1-15 | |
| X | DOMINIQUE THABUT ET AL: "Non-invasive diagnosis of large oesophageal varices with FibroTest in patients with cirrhosis: a preliminary retrospective study", LIVER INTERNATIONAL, vol. 26, no. 3, 1 April 2006 (2006-04-01), pages 271-278, XP055195113, ISSN: 1478-3223, DOI: 10.1111/j.1478-3231.2005.01227.x * abstract * | 1-15 | |
| X | K.C. THOMOPOULOS ET AL: "Non-invasive predictors of the presence of large oesophageal varices in patients with cirrhosis", DIGESTIVE AND LIVER DISEASE, vol. 35, no. 7, 1 July 2003 (2003-07-01), pages 473-478, XP055195117, ISSN: 1590-8658, DOI: 10.1016/S1590-8658(03)00219-6 * abstract * | 1-15 | **TECHNICAL FIELDS SEARCHED (IPC)** |
| A | EP 2 498 195 A1 (CT HOSPITALIER UNIVERSITAIRE D ANGERS [FR]; UNIV ANGERS [FR]) 12 September 2012 (2012-09-12) * the whole document * | 1-15 | |

-/--

The present search report has been drawn up for all claims

| Place of search | Date of completion of the search | Examiner |
|---|---|---|
| Munich | 17 June 2015 | Hinchliffe, Philippe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 15 16 2685

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JÉRÔME BOURSIER ET AL: "Improved diagnostic accuracy of blood tests for severe fibrosis and cirrhosis in chronic hepatitis C", EUROPEAN JOURNAL OF GASTROENTEROLOGY & HEPATOLOGY, vol. 21, no. 1, 1 January 2009 (2009-01-01), pages 28-38, XP055046919, ISSN: 0954-691X, DOI: 10.1097/MEG.0b013e32830cebd7 * see section 3.3.2 * | 1-15 | |
| T | "The international liver conference 2015", 22 April 2015 (2015-04-22), Elsevier, XP009184779, pages s201-s201, * abstract * | | |

TECHNICAL FIELDS
SEARCHED (IPC)

The present search report has been drawn up for all claims

| Place of search | Date of completion of the search | Examiner |
|---|---|---|
| Munich | 17 June 2015 | Hinchliffe, Philippe |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 15 16 2685

This annex lists the patent family members relating to the patent documents cited in the above-mentioned European search report.
The members are as contained in the European Patent Office EDP file on
The European Patent Office is in no way liable for these particulars which are merely given for the purpose of information.

17-06-2015

| Patent document cited in search report | Publication date | Patent family member(s) | Publication date |
|---|---|---|---|
| WO 2014190170 A1 | 27-11-2014 | NONE | |
| EP 2498195 A1 | 12-09-2012 | CN 103517668 A<br>EP 2498195 A1<br>EP 2684148 A1<br>JP 2014508936 A<br>US 2014005500 A1<br>WO 2012123428 A1 | 15-01-2014<br>12-09-2012<br>15-01-2014<br>10-04-2014<br>02-01-2014<br>20-09-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005116901 A
- WO 2014190170 A
- WO 03073822 A

**Non-patent literature cited in the description**

- **WAI et al.** *Hepatology,* 2003
- **STERLING et al.** *Hepatology,* 2006
- **ROSENBERG et al.** *Gastroenterology,* 2004
- **CALES et al.** *Hepatology,* 2005
- **PATEL et al.** *J Hepatology,* 2004
- **ADAMS et al.** *Clin Chem,* 2005
- **SACHER-HUVELIN S et al.** *Endoscopy,* 2015
- **OBERTI F et al.** *Gastrointest Endosc,* 1998, vol. 48, 148-157
- **PASCAL JP et al.** *N Engl J Med,* 1987, vol. 317, 856-861
- **CASTERA L et al.** *J Hepatol,* 2008, vol. 48, 835-847
- **ADAMS LA et al.** *Clin Chem,* 2005, vol. 51, 1867-1873
- **STERLING RK et al.** *Hepatology,* 2006, vol. 43, 1317-1325
- **WAI CT et al.** *Hepatology,* 2003, vol. 38, 518-526
- **LEROY V et al.** *Clin Biochem,* 2008, vol. 41, 1368-1376
- **BOURSIER J et al.** *Eur J Gastroenterol Hepatol,* 2009, vol. 21, 28-38
- **CALÈS P et al.** *J Hepatol,* 2010, vol. 52, S406
- **CALES P et al.** *Gastroenterol Clin Biol,* 2008, vol. 32, 40-51
- **CALES P et al.** *J Hepatol,* 2009, vol. 50, 165-173
- **CALES P et al.** *Liver Int,* 2010, vol. 30, 1346-1354
- **CALES P et al.** *Hepatology,* 2005, vol. 42, 1373-1381
- **CALES P et al.** *Liver international: official journal of the International Association for the Study of the Liver,* 2014, vol. 34, 907-917
- **BOSSUYT PM et al.** *Clin Chem,* 2003, vol. 49, 7-189
- **BOURSIER J et al.** *J Hepatol,* 2015
- **CALES P et al.** *Journal of clinical gastroenterology,* 2014
- **COLLI A et al.** *Cochrane Database Syst Rev,* 2014, vol. 10, CD008760
- **STEFANESCU H et al.** *Liver Int,* 2014
- **BUREAU C et al.** *Aliment Pharmacol Ther,* 2008, vol. 27, 1261-1268
- **CASTERA L et al.** *J Hepatol,* 2009, vol. 50, 59-68